# EUROPEAN PATENT APPLICATION

(11) **EP 3 806 174 A1**
(43) Date of publication of application: **14.04.2021**
(21) Application number: 19811483.7
(22) Date of filing: 23.05.2019
(51) Int. Cl.: H01L 51/42, C07D 495/04, H01L 27/30

(54) **PHOTOELECTRIC CONVERSION ELEMENT, IMAGING ELEMENT, OPTICAL SENSOR, AND COMPOUND**

(30) Priority: 31.05.2018 JP 2018105215; 27.11.2018 JP 2018221440
(71) Applicant: FUJIFILM Corporation, Tokyo 106-8620 (JP)
(72) Inventor: MASHIKO, Tomoyuki, Kanagawa 258-8577 (JP); YOSHIOKA, Tomoaki, Kanagawa 258-8577 (JP); FUKUZAKI, Eiji, Kanagawa 258-8577 (JP)
(74) Representative: Hoffmann Eitle
(86) International application number: PCT/JP2019/020499
(87) International publication number: WO 2019/230562

(57) **Abstract**

A first object of the present invention is to provide a photoelectric conversion element which includes a photoelectric conversion film having a narrow half-width of an absorption peak and has excellent heat resistance. Moreover, a second object of the present invention is to provide an imaging element and an optical sensor. Furthermore, a third object of the present invention is to provide a novel compound.

A photoelectric conversion element according to the embodiment of the present invention includes a conductive film, a photoelectric conversion film, and a transparent conductive film in this order, in which the photoelectric conversion film contains a compound represented by Formula (1).

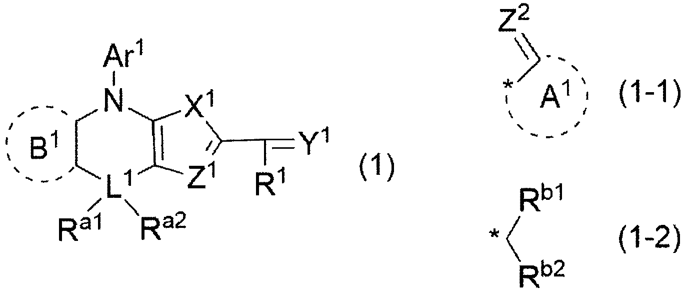

## Description

### BACKGROUND OF THE INVENTION

### 1. Field of the Invention

The present invention relates to a photoelectric conversion element, an imaging element, an optical sensor, and a compound.

### 2. Description of the Related Art

In recent years, development of an element (for example, an imaging element) having a photoelectric conversion film has progressed.

Regarding a photoelectric conversion element using a photoelectric conversion film, for example, JP2014-082483A discloses a photoelectric conversion element having a photoelectric conversion film containing a predetermined compound.

### SUMMARY OF THE INVENTION

As one aspect of an imaging element, there is a lamination-type imaging element in which a plurality of photoelectric conversion elements that receive different types of light are laminated. In a case where light is incident on the imaging element, a part of the incidence ray is absorbed by the photoelectric conversion elements arranged on the incident side, and the transmitted light is absorbed by the photoelectric conversion elements arranged further inside. In such an imaging element, since colors are easily separated, it is preferable that the absorption peak of each photoelectric conversion element has a narrow half-width.

The present inventors have examined the characteristics of the photoelectric conversion element described in JP2014-082483A, and as a result, have found that the half-width of the absorption peak of the photoelectric conversion film in the photoelectric conversion element is wide, and further improvement is necessary. Furthermore, it has been clarified that further improvement in the heat resistance of the photoelectric conversion element is also necessary.

In view of the circumstances, an object of the present invention is to provide a photoelectric conversion element which includes a photoelectric conversion film having a narrow half-width of an absorption peak and has excellent heat resistance.

Moreover, another object of the present invention is to provide an imaging element and an optical sensor.

Furthermore, still another object of the present invention is to provide a novel compound.

The present inventors have conducted extensive studies on the above-described objects, and as a result, have found that the above-described objects can be achieved by using, for the photoelectric conversion film, a compound having a predetermined structure, thereby completing the present invention.
[1] A photoelectric conversion element comprising, in the following order: a conductive film; a photoelectric conversion film; and a transparent conductive film, in which the photoelectric conversion film contains a compound represented by Formula (1).
[2] The photoelectric conversion element as described in [1], in which the compound represented by Formula (1) is a compound represented by Formula (2).
[3] The photoelectric conversion element as described in [2], in which R^{a5} and R^{a6} each independently represent an alkyl group having 3 or more carbon atoms, an aryl group, or a heteroaryl group.
[4] The photoelectric conversion element as described in [2] or [3], in which the compound represented by Formula (2) is a compound represented by Formula (2A).
[5] The photoelectric conversion element as described in [2], in which the compound represented by Formula (2) is a compound represented by Formula (3).
[6] The photoelectric conversion element as described in [5], in which R^{a5} and R^{a6} each independently represent an alkyl group having 3 or more carbon atoms, an aryl group, or a heteroaryl group.
[7] The photoelectric conversion element as described in [5] or [6], in which the compound represented by Formula (3) is a compound represented by Formula (3A).
[8] The photoelectric conversion element as described in any one of [2] to [4], in which B¹ represents an aromatic ring having a substituent, and at least one of the substituent that B¹ has or Ar¹ represents a group represented by Formula (4A), a group represented by Formula (4B), or a group represented by Formula (5).
[9] The photoelectric conversion element as described in [8], in which at least one of R^{e1}, ..., or R^{e4} in the group represented by Formula (4A) represents a substituent.
[10] The photoelectric conversion element as described in [8] or [9], in which Ar¹ represents the group represented by Formula (4A), the group represented by Formula (4B), or the group represented by Formula (5), and at least one of the substituents that B¹ has represents the group represented by Formula (4A) or the group represented by Formula (4B).
[11] The photoelectric conversion element as described in any one of [5] to [7], in which at least one of Ar¹, R³, ..., or R⁶ represents a group represented by Formula (4A), a group represented by Formula (4B), or a group represented by Formula (5).
[12] The photoelectric conversion element as described in [11], in which at least one of R^{e1}, ..., or R^{e4} in the group represented by Formula (4A) represents a substituent.
[13] The photoelectric conversion element as described in [11] or [12], in which Ar¹ represents the group represented by Formula (4A), the group represented by Formula (4B), or the group represented by Formula (5), and at least one of R³, ..., or R⁶ represents the group represented by Formula (4A) or the group represented by Formula (4B).
[14] The photoelectric conversion element as described in any one of [1] to [13], in which the photoelectric conversion film further contains an n-type organic semiconductor, and the photoelectric conversion film has a bulk hetero structure formed in a state where the compound represented by Formula (1) and the n-type organic semiconductor are mixed.
[15] The photoelectric conversion element as described in [14], in which the n-type organic semiconductor contains fullerenes selected from the group consisting of a fullerene and a derivative thereof.
[16] The photoelectric conversion element as described in any one of [1] to [15], further comprising one or more interlayers between the conductive film and the transparent conductive film, in addition to the photoelectric conversion film.
[17] An imaging element comprising the photoelectric conversion element as described in any one of [1] to [16].
[18] The imaging element as described in [17], further comprising another photoelectric conversion element which receives light having a wavelength different from a wavelength of light received by the photoelectric conversion element.
[19] The imaging element as described in [18], in which the photoelectric conversion element and the other photoelectric conversion element are laminated, and at least some of incidence rays are transmitted through the photoelectric conversion element, and then are received by the other photoelectric conversion element.
[20] The imaging element as described in [18] or [19], in which the photoelectric conversion element is a green photoelectric conversion element, and the other photoelectric conversion element includes a blue photoelectric conversion element and a red photoelectric conversion element.
[21] An optical sensor comprising the photoelectric conversion element as described in any one of [1] to [16].
[22] A compound represented by Formula (1).
[23] The compound as described in [22], in which the compound represented by Formula (1) is a compound represented by Formula (3).

According to the present invention, it is possible to provide a photoelectric conversion element including a photoelectric conversion film having a narrow half-width of an absorption peak and having excellent heat resistance.

Moreover, according to the present invention, it is possible to provide an imaging element and an optical sensor.

Furthermore, according to the present invention, it is possible to provide a novel compound.

### BRIEF DESCRIPTION OF THE DRAWINGS

Fig. 1 is a schematic cross-sectional view showing an example of a configuration of a photoelectric conversion element.
Fig. 2 is a schematic cross-sectional view showing an example of a configuration of a photoelectric conversion element.
Fig. 3 is a schematic cross-sectional view of one embodiment of an imaging element.
Fig. 4 is a ¹H NMR spectrum (400 MHz, CDCl₃) of a compound (D-6) in Example.
Fig. 5 is a ¹H NMR spectrum (400 MHz, CDCl₃) of a compound (D-2) in Example.
Fig. 6 is a ¹H NMR spectrum (400 MHz, CDCl₃) of a compound (D-4) in Example.
Fig. 7 is a ¹H NMR spectrum (400 MHz, CDCl₃) of a compound (D-7) in Example.
Fig. 8 is a ¹H NMR spectrum (400 MHz, CDCl₃) of a compound (D-8) in Example.
Fig. 9 is a ¹H NMR spectrum (400 MHz, CDCl₃) of a compound (D-9) in Example.
Fig. 10 is a ¹H NMR spectrum (400 MHz, CDCl₃) of a compound (D-12) in Example.
Fig. 11 is a ¹H NMR spectrum (400 MHz, CDCl₃) of a compound (D-13) in Example.
Fig. 12 is a ¹H NMR spectrum (400 MHz, CDCl₃) of a compound (D-15) in Example.

### DESCRIPTION OF THE PREFERRED EMBODIMENTS

### [Photoelectric conversion element]

Hereinafter, suitable embodiments of a photoelectric conversion element according to the embodiment of the present invention will be described.

In addition, in the present specification, a substituent for which whether it is substituted or unsubstituted is not specified may be further substituted with a substituent (for example, a substituent W described later) within the scope not impairing an intended effect. For example, the expression of an "alkyl group" refers to an alkyl group which may be substituted with a substituent (for example, the substituent W described later).

In addition, in the present specification, a numerical range expressed using "to" means a range including numerical values denoted before and after "to" as a lower limit value and an upper limit value.

In the present specification, a hydrogen atom may be a light hydrogen atom (an ordinary hydrogen atom) or a deuterium atom (a double hydrogen atom or the like).

A feature of the photoelectric conversion element according to the embodiment of the present invention is, for example, that a bulky substituent is introduced into a compound which is represented by Formula (1) (hereinafter, also referred to as a "specific compound") and contained in the photoelectric conversion film. More specifically, by introducing a bulky substituent at at least any position selected from positions of R^{a1} and R^{a2}, a position of Ar¹, and a position of B¹ in Formula (1), a structure of the specific compound is twisted, and association of the specific compounds in the photoelectric conversion film is suppressed. As a result, the half-width of the absorption peak of the photoelectric conversion film is narrowed, and the photoelectric conversion element is presumed to have excellent heat resistance.

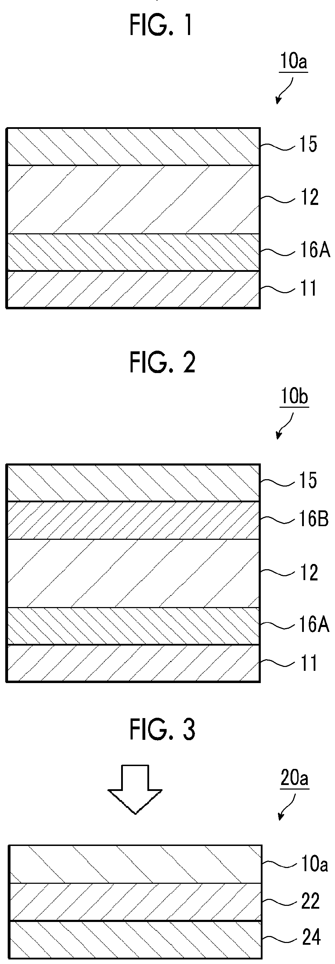

Fig. 1 shows a schematic cross-sectional view of the photoelectric conversion element according to one embodiment of the present invention.

A photoelectric conversion element 10a shown in Fig. 1 has a configuration in which a conductive film (hereinafter, also referred to as a lower electrode) 11 functioning as a lower electrode, an electron blocking film 16A, a photoelectric conversion film 12 containing the specific compound described later, and a transparent conductive film (hereinafter, also referred to as an upper electrode) 15 functioning as an upper electrode are laminated in this order.

Fig. 2 shows a configuration example of another photoelectric conversion element. A photoelectric conversion element 10b shown in Fig. 2 has a configuration in which the electron blocking film 16A, the photoelectric conversion film 12, a positive hole blocking film 16B, and the upper electrode 15 are laminated in this order on the lower electrode 11. Moreover, the lamination order of the electron blocking film 16A, the photoelectric conversion film 12, and the positive hole blocking film 16B in Figs. 1 and 2 may be appropriately changed according to the application and the characteristics.

In the photoelectric conversion element 10a (or 10b), it is preferable that light is incident on the photoelectric conversion film 12 through the upper electrode 15.

Moreover, in a case where the photoelectric conversion element 10a (or 10b) is used, a voltage can be applied. In this case, it is preferable that the lower electrode 11 and the upper electrode 15 form a pair of electrodes and a voltage of 1 × 10⁻⁵ to 1 × 10⁷ V/cm is applied between the pair of electrodes. From the viewpoint of performance and power consumption, the voltage to be applied is more preferably 1 × 10⁻⁴ to 1 × 10⁷ V/cm and still more preferably 1 × 10⁻³ to 5 × 10⁶ V/cm.

Furthermore, regarding the voltage application method, it is preferable that the voltage is applied such that the electron blocking film 16A side serves as a cathode and the photoelectric conversion film 12 side serves as an anode, in Figs. 1 and 2. Even in a case where the photoelectric conversion element 10a (or 10b) is used as an optical sensor, or a case where the photoelectric conversion element 10a (or 10b) is incorporated in an imaging element, the voltage can be applied by the same method.

As will be described in detail later, the photoelectric conversion element 10a (or 10b) can be suitably applied to applications of the imaging element.

Hereinafter, a form of each layer constituting the photoelectric conversion element according to the embodiment of the present invention will be described in detail.

### <Photoelectric conversion film>

The photoelectric conversion film is a film containing the specific compound as a photoelectric conversion material. By using the compound, a photoelectric conversion element including a photoelectric conversion film having a narrow half-width of an absorption peak and having excellent heat resistance can be obtained.

Hereinafter, the specific compound will be described in detail.

Formula (1) includes all geometric isomers which can be distinguished based on a C=C double bond constituted by a carbon atom to which R¹ is bonded and a carbon atom adjacent to the carbon atom in Formula (1). That is, both a cis isomer and a trans isomer which are distinguished based on the C=C double bond are included in Formula (1).

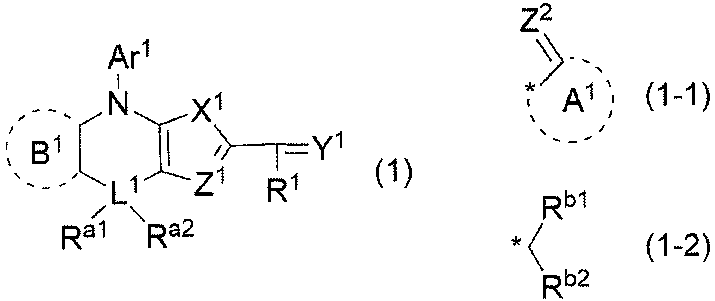

In Formula (1), Ar¹ represents an aryl group which may have a substituent or a heteroaryl group which may have a substituent.

The number of carbon atoms in the aryl group is not particularly limited, but is preferably 6 to 30, more preferably 6 to 18, and still more preferably 6 to 12. The aryl group may have a monocyclic structure or a condensed ring structure (a fused ring structure) in which two or more rings are condensed.

As the aryl group, for example, a phenyl group, a naphthyl group, or an anthryl group is preferable, and a phenyl group or a naphthyl group is more preferable.

Examples of the substituent that the aryl group may have include the substituent W described later, and examples thereof include an alkyl group.

The aryl group may have a plurality of types of substituents.

In a case where the aryl group has a substituent, the number of substituents that the aryl group has is not particularly limited, but is preferably 1 to 5 from the viewpoint that the half-width of the absorption peak of the photoelectric conversion film is narrower and/or the heat resistance of the photoelectric conversion element is superior (hereinafter, also simply referred to as the "viewpoint that the effect of the present invention is superior"). Moreover, in a case where the aryl group represents a phenyl group, the number of substituents that the phenyl group has is more preferably 2 to 5 from the viewpoint that the effect of the present invention is superior.

The number of carbon atoms in the heteroaryl group (a monovalent aromatic heterocyclic group) is not particularly limited, but is preferably 3 to 30 and more preferably 3 to 18.

The heteroaryl group has a carbon atom, and a hetero atom other than a hydrogen atom. Examples of the hetero atom include a sulfur atom, an oxygen atom, a nitrogen atom, a selenium atom, a tellurium atom, a phosphorus atom, a silicon atom, and a boron atom, and a sulfur atom, an oxygen atom, or a nitrogen atom is preferable.

The number of hetero atoms that the heteroaryl group has is not particularly limited, but is preferably 1 to 10, more preferably 1 to 4, and still more preferably 1 or 2.

The number of ring members of the heteroaryl group is not particularly limited, but is preferably 3 to 8, more preferably 5 to 7, and still more preferably 5 or 6. Moreover, the heteroaryl group may have a monocyclic structure or a condensed ring structure in which two or more rings are condensed. In a case of the condensed ring structure, an aromatic hydrocarbon ring (for example, a benzene ring) having no hetero atom may be included.

Examples of the heteroaryl group include a furyl group, a pyridyl group, a quinolyl group, an isoquinolyl group, an acridinyl group, a phenanthridinyl group, a pteridinyl group, a pyrazinyl group, a quinoxalinyl group, a pyrimidinyl group, a quinazolyl group, a pyridazinyl group, a cinnolinyl group, a phthalazinyl group, a triazinyl group, an oxazolyl group, a benzoxazolyl group, a thiazolyl group, a benzothiazolyl group, an imidazolyl group, a benzimidazolyl group, a pyrazolyl group, an indazolyl group, an isoxazolyl group, a benzisoxazolyl group, an isothiazolyl group, a benzisothiazolyl group, an oxadiazolyl group, a thiadiazolyl group, a triazolyl group, a tetrazolyl group, a benzofuryl group, a thienyl group, a benzothienyl group, a dibenzofuryl group, a dibenzothienyl group, a pyrrolyl group, an indolyl group, an imidazopyridinyl group, and a carbazolyl group.

Among them, a furyl group, a thienyl group, a pyridyl group, a quinolyl group, an isoquinolyl group, or a carbazolyl group is preferable.

Examples of the substituent that the heteroaryl group may have include the same substituents as the above-described substituents that the aryl group may have.

In a case where the heteroaryl group has a substituent, the number of substituents that the heteroaryl group has is not particularly limited, but is preferably 1 to 5.

As Ar¹, among them, a group represented by Formula (1-3) is preferable from the viewpoint that the effect of the present invention is superior.

R¹ represents a hydrogen atom or a substituent. Among them, R¹ is preferably a hydrogen atom from the viewpoint that the effect of the present invention is superior.

The definition of the substituent is the same as the definition of the substituent W described later. Examples of the substituent include an alkyl group, an aryl group, and a heteroaryl group.

X¹ represents a sulfur atom, an oxygen atom, a selenium atom, a tellurium atom, S(=O), S(=O)₂, NR^{c1}, SiR^{c2}R^{c3}, or CR^{c4}R^{c5}.

R^{c1} to R^{c5} each independently represent a hydrogen atom or a substituent. The definition of the substituent is the same as the definition of the substituent W described later. Among them, examples of the substituent include an alkyl group, an aryl group, and a heteroaryl group.

X¹ is preferably a sulfur atom, an oxygen atom, or a selenium atom from the viewpoint that the effect of the present invention is superior.

Z¹ represents CR^{c6}(=CR^{c6}-) or a nitrogen atom (=N-). R^{c6} represents a hydrogen atom or a substituent. The definition of the substituent is the same as the definition of the substituent W described later. Among them, examples of the substituent include an alkyl group, an aryl group, and a heteroaryl group.

Z¹ is preferably CR^{c6} from the viewpoint that the effect of the present invention is superior.

R^{c6} is preferably a hydrogen atom.

L¹ represents a carbon atom, a silicon atom, or a germanium atom. Among them, a carbon atom is preferable from the viewpoint that the effect of the present invention is superior.

B¹ represents an aromatic ring which may have a substituent.

The aromatic ring may be monocyclic or polycyclic.

Examples of the aromatic ring include an aromatic hydrocarbon ring and an aromatic heterocyclic ring. Examples of the aromatic hydrocarbon ring include a benzene ring, a naphthalene ring, an anthracene ring, and a phenanthrene ring. Examples of the aromatic heterocyclic ring include a pyrrole ring, a furan ring, a thiophene ring, an imidazole ring, and an oxazole ring.

Among them, from the viewpoint that the effect of the present invention is superior, an aromatic hydrocarbon ring is preferable and a benzene ring is more preferable.

The definition of the substituent is the same as the definition of the substituent W described later, and examples of the substituent include an alkyl group, a silyl group, an aryl group, and a heteroaryl group. These groups may further have a substituent.

The number of carbon atoms in the alkyl group is preferably 1 to 12, more preferably 1 to 6, and still more preferably 1 to 3.

As the silyl group, for example, a group represented by -Si(R^{p})(R^{q})(R^{r}) is preferable. R^{p} to R^{r} each independently represent a hydrogen atom or a substituent. Examples of the substituent represented by R^{p} to R^{r} include an alkyl group (the alkyl group may be any of linear, branched, or cyclic, and the number of carbon atoms is preferably 1 to 4 and more preferably 1), an aryl group, and a heteroaryl group. These groups may further have a substituent.

Furthermore, examples of the aryl group and the heteroaryl group which are represented by R^{p} to R^{r} include the groups exemplified as the aryl group and the heteroaryl group which are represented by Ar¹.

Examples of the aryl group and the heteroaryl group include the groups exemplified as the aryl group and the heteroaryl group which are represented by Ar¹.

As the substituent that B¹ has, among them, from the viewpoint that the effect of the present invention is superior, an alkyl group, a silyl group, or a group represented by Formula (1-3) is more preferable, and a group represented by Formula (1-3) is still more preferable.

Y¹ represents a group represented by Formula (1-1) or a group represented by Formula (1-2). Among them, a group represented by Formula (1-1) is preferable from the viewpoint that the effect of the present invention is superior. * in Formula (1-1) and Formula (1-2) represents a bonding position.

A¹ represents a ring containing at least two carbon atoms. Moreover, the two carbon atoms refer to a carbon atom in a carbonyl group specified in Formula (1-1) and a carbon atom which is adjacent to the carbon atom in the carbonyl group and specified in Formula (1-1), and both the carbon atoms are atoms constituting A¹.

In addition, in the ring, carbon atoms constituting the ring may be substituted with another carbonyl carbon (>C=O) or thiocarbonyl carbon (>C=S). Furthermore, the other carbonyl carbon (>C=O) here refers to carbonyl carbon (excluding the carbonyl carbon specified in Formula (1-1)) that the carbon atoms constituting the ring have.

The number of carbon atoms in A¹ is preferably 3 to 30, more preferably 3 to 20, and still more preferably 3 to 15. Moreover, the number of carbon atoms is a number including two carbon atoms specified in the formula.

A¹ may have a hetero atom, examples thereof include a nitrogen atom, a sulfur atom, an oxygen atom, a selenium atom, a tellurium atom, a phosphorus atom, a silicon atom, and a boron atom, a nitrogen atom, a sulfur atom, or an oxygen atom is preferable, and an oxygen atom is more preferable.

The number of hetero atoms in A¹ is preferably 0 to 10, more preferably 0 to 5, and still more preferably 0 to 2. Moreover, the number of hetero atoms is a number which does not include the number of hetero atoms introduced into the ring by substituting carbon atoms constituting the ring represented by A¹ with carbonyl carbon (>C=O) or thiocarbonyl carbon (>C=S) (here, the carbonyl carbon (>C=O) is intended to include the carbonyl carbon specified in Formula (1-1)), and the number of hetero atoms that the substituent of A¹ has.

A¹ may have a substituent, and the substituent is preferably a halogen atom (preferably a chlorine atom), an alkyl group (the alkyl group may be any of linear, branched, or cyclic, and the number of carbon atoms is preferably 1 to 10 and more preferably 1 to 6), an aryl group (the number of carbon atoms is preferably 6 to 18 and more preferably 6 to 12), a heteroaryl group (the number of carbon atoms is preferably 5 to 18 and more preferably 5 or 6), or a silyl group (examples thereof include an alkylsilyl group, an alkyl group in the alkylsilyl group may be any of linear, branched, or cyclic, and the number of carbon atoms is preferably 1 to 4 and more preferably 1).

A¹ may or may not exhibit aromaticity.

A¹ may have a monocyclic structure or a condensed ring structure, but is preferably a 5-membered ring, a 6-membered ring, or a fused ring containing at least any one of a 5-membered ring or a 6-membered ring. The number of rings forming the fused ring is preferably 1 to 4 and more preferably 1 to 3.

As the ring represented by A¹, a ring usually used as an acidic nucleus (specifically, an acidic nucleus in a merocyanine dye) is preferable, and specific examples thereof as follows.
(a) 1,3-Dicarbonyl nucleus: for example, 1,3-indandione nucleus, 1,3-cyclohexanedione, 5,5-dimethyl-1,3-cyclohexanedione, 1,3-dioxane-4,6-dione, and the like.
(b) Pyrazolinone nucleus: for example, 1-phenyl-2-pyrazolin-5-one, 3-methyl-1-phenyl-2-pyrazolin-5-one, 1-(2-benzothiazolyl)-3-methyl-2-pyrazolin-5-one, and the like.
(c) Isoxazolinone nucleus: for example, 3-phenyl-2-isoxazolin-5-one, 3-methyl-2-isoxazolin-5-one, and the like.
(d) Oxindole nucleus: for example, 1-alkyl-2,3-dihydro-2-oxindole, and the like.
(e) 2,4,6-Trioxohexahydropyrimidine nucleus: for example, barbituric acid or 2-thiobarbituric acid, a derivative thereof, and the like. Examples of the derivative include a 1-alkyl form such as 1-methyl and 1-ethyl, a 1,3-dialkyl form such as 1,3-dimethyl, 1,3-diethyl, and 1,3-dibutyl, a 1,3-diaryl form such as 1,3-diphenyl, 1,3-di(p-chlorophenyl), and 1,3-di(p-ethoxycarbonylphenyl), a 1-alkyl-1-aryl form such as 1-ethyl-3-phenyl, and a 1,3-diheteroaryl form such as 1,3-di(2-pyridyl).
(f) 2-Thio-2,4-thiazolidinedione nucleus: for example, rhodanine, a derivative thereof, and the like. Examples of the derivative include 3-alkylrhodanine such as 3-methylrhodanine, 3-ethylrhodanine, and 3-allylrhodanine, 3-arylrhodanine such as 3-phenylrhodanine, and 3-heteroaryl rhodanine such as 3-(2-pyridyl) rhodanine.
(g) 2-Thio-2,4-oxazolidinedione nucleus (2-thio-2,4-(3H,5H)-oxazoledione nucleus): for example, 3-ethyl-2-thio-2,4-oxazolidinedione, and the like.
(h) Thianaphthenone nucleus: for example, 3(2H)-thianaphthenone-1,1-dioxide, and the like.
(i) 2-Thio-2,5-thiazolidinedione nucleus: for example, 3-ethyl-2-thio-2,5-thiazolidinedione, and the like.
(j) 2,4-Thiazolidinedione nucleus: for example, 2,4-thiazolidinedione, 3-ethyl-2,4-thiazolidinedione, 3-phenyl-2,4-thiazolidinedione, and the like.
(k) Thiazolin-4-one nucleus: for example, 4-thiazolinone, 2-ethyl-4-thiazolinone, and the like.
(l) 2,4-Imidazolidinedione (hydantoin) nucleus: for example, 2,4-imidazolidinedione, 3-ethyl-2,4-imidazolidinedione, and the like.
(m) 2-Thio-2,4-imidazolidinedione (2-thiohydantoin) nucleus: for example, 2-thio-2,4-imidazolidinedione, 3-ethyl-2-thio-2,4-imidazolidinedione, and the like.
(n) Imidazolin-5-one nucleus: for example, 2-propylmercapto-2-imidazolin-5-one, and the like.
(o) 3,5-Pyrazolidinedione nucleus: for example, 1,2-diphenyl-3,5-pyrazolidinedione, 1,2-dimethyl-3,5-pyrazolidinedione, and the like.
(p) Benzothiophen-3(2H)-one nucleus: for example, benzothiophen-3(2H)-one, oxobenzothiophen-3(2H)-one, dioxobenzothiophen-3(2H)-one, and the like.
(q) Indanone nucleus: for example, 1-indanone, 3-phenyl-1-indanone, 3-methyl-1-indanone, 3,3-diphenyl-1-indanone, 3,3-dimethyl-1-indanone, and the like.
(r) Benzofuran-3-(2H)-one nucleus: for example, benzofuran-3-(2H)-one and the like.
(s) 2,2-Dihydrophenalene-1,3-dione nucleus and the like.

In Formula (1-1), Z² represents an oxygen atom, a sulfur atom, =NR^{Z1}, or =CR^{Z2}R^{Z3}.

R^{Z1} represents a hydrogen atom or a substituent. R^{Z2} and R^{Z3} each independently represent a cyano group or -COOR^{Z4}.

R^{Z4} represents an alkyl group which may have a substituent, an aryl group which may have a substituent, or a heteroaryl group which may have a substituent.

Z² is preferably an oxygen atom.

R^{b1} and R^{b2} each independently represent a cyano group or -COOR^{c7}.

R^{c7} represents an alkyl group or an aryl group.

R^{a1} and R^{a2} each independently represent a hydrogen atom or a substituent. The definition of the substituent is the same as the definition of the substituent W described later. Among them, examples of the substituent include an alkyl group, an aryl group, and a heteroaryl group.

The alkyl group is preferably an alkyl group having 3 or more carbon atoms from the viewpoint that the effect of the present invention is superior. The upper limit of the number of carbon atoms is not particularly limited, but is 10 or less, for example.

The alkyl group is more preferably a secondary alkyl group having 3 or more carbon atoms. Moreover, the secondary alkyl group refers to an alkyl group having a secondary carbon atom.

Examples of the secondary alkyl group having 3 or more carbon atoms include an isopropyl group, an isobutyl group, a pentan-2-yl group, a pentan-3-yl group, and a 3-methyl-2-pentyl group.

Examples of the aryl group include the groups exemplified as the aryl group represented by Ar¹.

Examples of the heteroaryl group include the groups exemplified as the heteroaryl group represented by Ar¹.

R^{a1} and R^{a2} may be bonded to each other to form a ring. More specifically, R^{a1} and R^{a2} may be bonded to each other via a single bond or a divalent linking group to form a ring.

Examples of the divalent linking group include -O-, -S-, an alkylene group, a silylene group, an alkenylene group, a cycloalkylene group, a cycloalkenylene group, an arylene group, a divalent heterocyclic group, and an imino group.

Examples of the ring formed by bonding R^{a1} and R^{a2} to each other include an aromatic ring (an aromatic hydrocarbon ring or an aromatic heterocyclic ring) and a non-aromatic ring.

Examples of the aromatic ring include a benzene ring and a fluorene ring.

In addition, in a case where R^{a1} and R^{a2} are bonded to each other to form a ring, the specific compound is preferably a compound represented by Formula (1A).

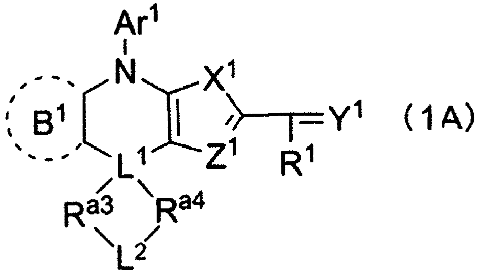

The definitions of Ar¹, R¹, X¹, Z¹, L¹, B¹, and Y¹ in Formula (1A) are the same as the definitions of Ar¹, R¹, X¹, Z¹, L¹, B¹, and Y¹ in Formula (1), and preferred aspects thereof are also the same.

R^{a3} and R^{a4} each independently represent an arylene group or a heteroarylene group. Moreover, the arylene group or heteroarylene group represented by R^{a3} and R^{a4} may have a substituent (for example, a group exemplified as the substituent W).

The arylene group is more preferably a phenylene group.

The number of carbon atoms in the heteroarylene group (a divalent aromatic heterocyclic group) is not particularly limited, but is preferably 3 to 30 and more preferably 3 to 18.

The heteroarylene group has a carbon atom, and a hetero atom other than a hydrogen atom. Examples of the hetero atom include a sulfur atom, an oxygen atom, a nitrogen atom, a selenium atom, a tellurium atom, a phosphorus atom, a silicon atom, and a boron atom, and a sulfur atom, an oxygen atom, or a nitrogen atom is preferable.

The number of hetero atoms that the heteroarylene group has is not particularly limited, but is preferably 1 to 10, more preferably 1 to 4, and still more preferably 1 or 2.

The number of ring members of the heteroarylene group is not particularly limited, but is preferably 5 to 8, more preferably 5 to 7, and still more preferably 5 or 6. Moreover, the heteroarylene group may have a monocyclic structure or a condensed ring structure in which two or more rings are condensed. In a case of the condensed ring structure, an aromatic hydrocarbon ring (for example, a benzene ring) having no hetero atom may be included.

L² represents a single bond or a divalent linking group.

Examples of the divalent linking group include -O-, -S-, an alkylene group, a silylene group, an alkenylene group, a cycloalkylene group, a cycloalkenylene group, an arylene group, a divalent heterocyclic group, and an imino group.

In a case where both R^{a1} and R^{a2} in Formula (1) are selected from the group consisting of a hydrogen atom and a methyl group, the compound represented by Formula (1) satisfies the following condition (1a) or the following condition (1b).

Condition (1a): Ar¹ represents a group represented by Formula (1-3).

Condition (1b): B¹ represents an aromatic ring having a substituent selected from the group consisting of an alkyl group, a silyl group, and a group represented by Formula (1-3).

The group represented by Formula (1-3) is shown below.

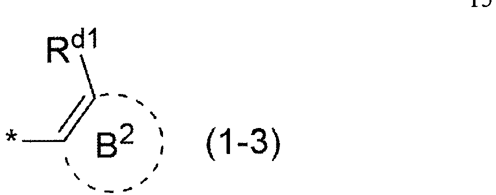

In Formula (1-3), B² represents an aromatic ring which may have a substituent.

The definition of the aromatic ring which may have a substituent and is represented by B² is the same as the definition of the aromatic ring which may have a substituent and is represented by B¹, and preferred aspects thereof are also the same.

R^{dl} represents a hydrogen atom or a substituent selected from the group consisting of an alkyl group, a silyl group, an alkoxy group, an alkylthio group, a cyano group, a halogen atom, an aryl group, a heteroaryl group, an alkenyl group, and an alkynyl group.

The number of carbon atoms in the alkyl group represented by R^{dl} is preferably 1 to 12, more preferably 1 to 6, and still more preferably 1 to 3.

Examples of the silyl group represented by R^{dl} include the silyl group described as the substituent of the aromatic ring which may have a substituent and is represented by B¹.

The number of carbon atoms in the alkoxy group represented by R^{d1} is preferably 1 to 12, more preferably 1 to 6, and still more preferably 1 to 3.

The number of carbon atoms in the alkylthio group represented by R^{dl} is preferably 1 to 12, more preferably 1 to 6, and still more preferably 1 to 3.

Examples of the halogen atom represented by R^{dl} include a fluorine atom, an iodine atom, a bromine atom, and a chlorine atom.

Examples of the aryl group represented by R^{dl} include the groups exemplified as the aryl group represented by Ar¹.

Examples of the heteroaryl group represented by R^{dl} include the groups exemplified as the heteroaryl group represented by Ar¹.

The number of carbon atoms in the alkenyl group represented by R^{dl} is preferably 2 to 12, more preferably 2 to 6, and still more preferably 2 or 3.

The number of carbon atoms in the alkynyl group represented by R^{dl} is preferably 2 to 12, more preferably 2 to 6, and still more preferably 2 or 3.

However, in a case where B² represents a benzene ring which may have a substituent, R^{d1} represents a group other than a hydrogen atom. That is, in a case where B² represents a benzene ring which may have a substituent, R^{dl} represents a substituent selected from the group consisting of an alkyl group, a silyl group, an alkoxy group, an alkylthio group, a cyano group, a halogen atom, an aryl group, a heteroaryl group, an alkenyl group, and an alkynyl group, and among them, an alkyl group or a halogen atom is preferable and an alkyl group is more preferable.

In addition, the substituent represented by R^{dl} may further have a substituent (examples of the substituent include the groups (for example, a halogen atom such as a fluorine atom) exemplified as the substituent W).

The substituents that R^{dl} and B² have may be bonded to each other to form a non-aromatic ring.
* represents a bonding position.

As the group represented by Formula (1-3), among them, from the viewpoint that the effect of the present invention is superior, a group represented by Formula (4A), a group represented by Formula (4B), or a group represented by Formula (5) is preferable.

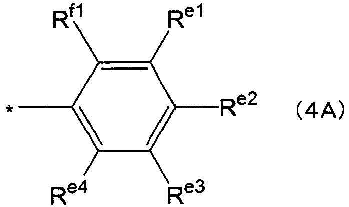

In Formula (4A), R^{e1} to R^{e4} each independently represent a hydrogen atom or a substituent.

From the viewpoint that the effect of the present invention is superior, it is preferable that at least one of R^{e1}, ..., or R^{e4} represents a substituent, and more preferable that at least two of R^{e1}, ..., or R^{e4} each represent a substituent.

The definition of the substituent is the same as the definition of the substituent W described later. Examples of the substituent include an alkyl group, an aryl group, a heteroaryl group, a silyl group, a halogen atom, and a cyano group. Moreover, these groups may further have a substituent (examples thereof include a halogen atom such as a fluorine atom).

The number of carbon atoms in each alkyl group represented by R^{e1} to R^{e4} is preferably 1 to 12, more preferably 1 to 6, and still more preferably 1 to 3.

Examples of the aryl groups represented by R^{e1} to R^{e4} include the groups exemplified as the aryl group represented by Ar¹.

Examples of the heteroaryl groups represented by R^{e1} to R^{e4} include the groups exemplified as the heteroaryl group represented by Ar¹.

Examples of the silyl groups represented by R^{e1} to R^{e4} include the silyl group described as the substituent of the aromatic ring which may have a substituent and is represented by B¹.

Examples of the halogen atoms represented by R^{e1} to R^{e4} include a fluorine atom, an iodine atom, a bromine atom, and a chlorine atom.

Furthermore, R^{e1} to R^{e4} may be bonded to each other to form a ring. Examples of the ring formed by bonding R^{e1} to R^{e4} to each other include an aromatic ring (an aromatic hydrocarbon ring or an aromatic heterocyclic ring) and a non-aromatic ring.

R^{f1} represents an alkyl group, a cyano group, or a halogen atom, and among them, an alkyl group is more preferable.

The number of carbon atoms in the alkyl group represented by R^{f1} is preferably 1 to 12, more preferably 1 to 6, and still more preferably 1 to 3. Moreover, the alkyl group may further have a substituent (examples thereof include a halogen atom such as a fluorine atom).

The halogen atom represented by R^{f1} is preferably a fluorine atom.

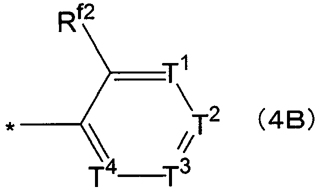

In Formula (4B), T¹ to T⁴ each independently represent CR^{e12}(=CR^{e12}-) or a nitrogen atom (=N-). R^{e12} represents a hydrogen atom or a substituent. Provided that at least one of T¹, ..., or T⁴ represents a nitrogen atom.

From the viewpoint that the effect of the present invention is superior, it is preferable that at least one of T¹, ..., or T⁴ represents CR^{e12} and at least one R^{e12} represents a substituent, and more preferable that at least two of T¹, ..., or T⁴ each represent CR^{e12} and at least two R^{e12}'s each represent a substituent.

The definition of the substituent is the same as the definition of the substituent W described later. Examples of the substituent include an alkyl group, an aryl group, a heteroaryl group, a silyl group, a halogen atom, and a cyano group. Moreover, these groups may further have a substituent (examples thereof include a halogen atom such as a fluorine atom).

The number of carbon atoms in the alkyl group represented by R^{e12} is preferably 1 to 12, more preferably 1 to 6, and still more preferably 1 to 3.

Examples of the aryl group represented by R^{e12} include the groups exemplified as the aryl group represented by Ar¹.

Examples of the heteroaryl group represented by R^{e12} include the groups exemplified as the heteroaryl group represented by Ar¹.

Examples of the silyl group represented by R^{e12} include the silyl group described as the substituent of the aromatic ring which may have a substituent and is represented by B¹.

Examples of the halogen atom represented by R^{e12} include a fluorine atom, an iodine atom, a bromine atom, and a chlorine atom.

Furthermore, in a case where a plurality of R^{e12}'s are present in Formula (4B), R^{e12}'s may be the same as or different from each other, and R^{e12}'s may be bonded to each other to form a ring. Examples of the ring formed by bonding R^{e12}'s to each other include an aromatic ring (an aromatic hydrocarbon ring or an aromatic heterocyclic ring) and a non-aromatic ring.

R^{f2} represents an alkyl group, a cyano group, or a halogen atom.

The number of carbon atoms in the alkyl group represented by R^{f2} is preferably 1 to 12, more preferably 1 to 6, and still more preferably 1 to 3. Moreover, the alkyl group may further have a substituent (examples thereof include a halogen atom such as a fluorine atom).

The halogen atom represented by R^{f2} is preferably a fluorine atom.

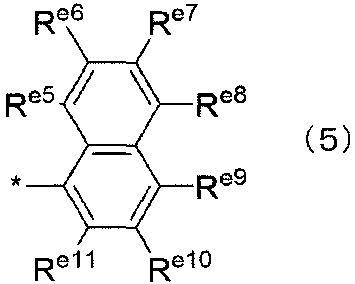

In Formula (5), R^{e5} to R^{e11} each independently represent a hydrogen atom or a substituent.

The definition of each substituent represented by R^{e5} to R^{e11} is the same as the definition of the substituent W described later. Examples of the substituent include an alkyl group, an aryl group, a heteroaryl group, a halogen atom, and a cyano group. Moreover, these groups may further have a substituent.

Examples of the alkyl groups represented by R^{e5} to R^{e11} include the groups exemplified as the alkyl groups represented by R^{e1} to R^{e4}.

Examples of the aryl groups represented by R^{e5} to R^{e11} include the groups exemplified as the aryl group represented by Ar¹.

Examples of the heteroaryl groups represented by R^{e5} to R^{e11} include the groups exemplified as the heteroaryl group represented by Ar¹.

Examples of the halogen atoms represented by R^{e5} to R^{e11} include a fluorine atom, an iodine atom, a bromine atom, and a chlorine atom.

Furthermore, R^{e5} to R^{e11} may be bonded to each other to form a ring. Examples of the ring formed by bonding R^{e5} to R^{e11} to each other include an aromatic ring (an aromatic hydrocarbon ring or an aromatic heterocyclic ring) and a non-aromatic ring.

Among them, R^{e5} to R^{e11} each are preferably a hydrogen atom, an alkyl group, or a halogen atom.

In Formula (1), from the viewpoint that the effect of the present invention is superior, it is preferable that B¹ represents an aromatic ring having a substituent and at least one of the substituent that B¹ has or Ar¹ represents the group represented by Formula (4A), the group represented by Formula (4B), or the group represented by Formula (5), and more preferable that Ar¹ represents the group represented by Formula (4A), the group represented by Formula (4B), or the group represented by Formula (5) and at least one of the substituents that B¹ has represents the group represented by Formula (4A) or the group represented by Formula (4B).

From the viewpoint that the effect of the present invention is superior, the specific compound is preferably a compound represented by Formula (2).

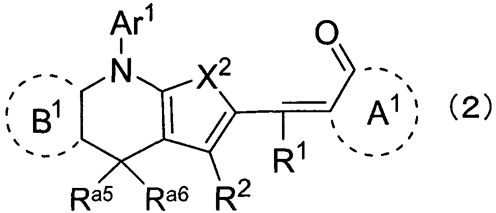

The definitions of Ar¹, R¹, B¹, and A¹ in Formula (2) are the same as the definitions of Ar¹, R¹, B¹, and A¹ in Formula (1), and preferred aspects thereof are also the same.

R² represents a hydrogen atom or a substituent.

The definition of the substituent represented by R² is the same as the definition of the substituent represented by R¹, and preferred aspects thereof are also the same.

X² represents a sulfur atom, an oxygen atom, or a selenium atom, and a sulfur atom is preferable.

R^{a5} and R^{a6} each independently represent a hydrogen atom, an alkyl group, an aryl group, or a heteroaryl group.

The definitions of the alkyl group, the aryl group, and the heteroaryl group which are represented by R^{a5} and R^{a6} are the same as the definitions of the alkyl group, the aryl group, and the heteroaryl group which are represented by R^{a1} and R^{a2} in Formula (1), and preferred aspects thereof are also the same.

In a case where both R^{a5} and R^{a6} in Formula (2) are selected from the group consisting of a hydrogen atom and a methyl group, the compound represented by Formula (2) satisfies the following condition (2a) or the following condition (2b).

Condition (2a): Ar¹ represents a group represented by Formula (1-3).

Condition (2b): B¹ represents an aromatic ring having a substituent selected from the group consisting of an alkyl group, a silyl group, and a group represented by Formula (1-3).

Furthermore, the group represented by Formula (1-3) is as described above.

In addition, R^{a5} and R^{a6} may be bonded to each other to form a ring. More specifically, R^{a5} and R^{a6} may be bonded to each other via a single bond or a divalent linking group to form a ring.

Examples of the divalent linking group include -O-, -S-, an alkylene group, a silylene group, an alkenylene group, a cycloalkylene group, a cycloalkenylene group, an arylene group, a divalent heterocyclic group, and an imino group.

Examples of the ring formed by bonding R^{a5} and R^{a6} to each other include an aromatic ring (an aromatic hydrocarbon ring or an aromatic heterocyclic ring) and a non-aromatic ring.

Examples of the aromatic ring include a benzene ring and a fluorene ring.

Furthermore, in a case where R^{a5} and R^{a6} are bonded to each other to form a ring, the compound represented by Formula (2) is preferably a compound represented by Formula (2A).

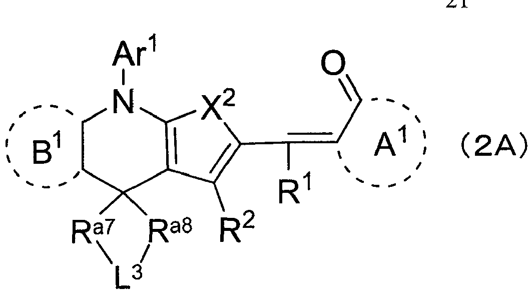

The definitions of Ar¹, R¹, R², X², B¹, and A¹ in Formula (2A) are the same as the definitions of Ar¹, R¹, R², X², B¹, and A¹ in Formula (2), and preferred aspects thereof are also the same.

R^{a7} and R^{a8} each independently represent an arylene group or a heteroarylene group.

The definition of the arylene group or heteroarylene group represented by R^{a7} and R^{a8} is the same as the definition of the arylene group or heteroarylene group represented by R^{a3} and R^{a4} in Formula (1A), and preferred aspects thereof are also the same.

L³ represents a single bond or a divalent linking group.

The definition of the divalent linking group represented by L³ is the same as the definition of the divalent linking group represented by L² in Formula (1A), and preferred aspects thereof are also the same.

In Formula (2) and Formula (2A), from the viewpoint that the effect of the present invention is superior, it is preferable that B¹ represents an aromatic ring having a substituent and at least one of the substituent that B¹ has or Ar¹ represents the group represented by Formula (4A), the group represented by Formula (4B), or the group represented by Formula (5), and more preferable that Ar¹ represents the group represented by Formula (4A), the group represented by Formula (4B), or the group represented by Formula (5) and at least one of the substituents that B¹ has represents the group represented by Formula (4A) or the group represented by Formula (4B).

From the viewpoint that the effect of the present invention is superior, the compound represented by Formula (2) is preferably a compound represented by Formula (3).

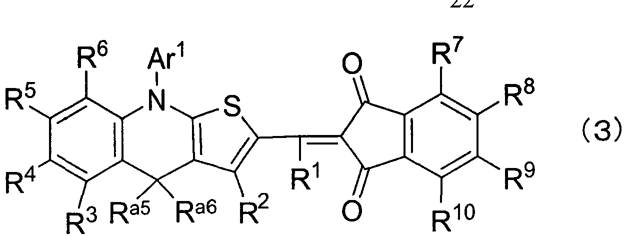

The definitions of Ar¹, R¹, R², R^{a5}, and R^{a6} in Formula (3) are the same as the definitions of Ar¹, R¹, R², R^{a5}, and R^{a6} in Formula (2), and preferred aspects thereof are also the same.

R³ to R¹⁰ each independently represent a hydrogen atom or a substituent.

The definition of each substituent represented by R³ to R¹⁰ is the same as the definition of the substituent of the aromatic ring which may have the substituent and is represented by B¹ in Formula (1).

R⁷ and R⁸, R⁸ and R⁹, and R⁹ and R¹⁰ may be each independently bonded to each other to form a ring. Examples of the type of the ring to be formed include an aromatic ring (an aromatic hydrocarbon ring or an aromatic heterocyclic ring) and a non-aromatic ring. Examples of the aromatic ring include a benzene ring and a fluorene ring.

In a case where both R^{a5} and R^{a6} in Formula (3) are selected from the group consisting of a hydrogen atom and a methyl group, the compound represented by Formula (3) satisfies the following condition (3a) or the following condition (3b).

Condition (3a): Ar¹ represents a group represented by Formula (1-3).

Condition (3b): B¹ represents an aromatic ring having a substituent selected from the group consisting of an alkyl group, a silyl group, and a group represented by Formula (1-3).

Furthermore, the group represented by Formula (1-3) is as described above.

In addition, R^{a5} and R^{a6} may be bonded to each other to form a ring. More specifically, R^{a5} and R^{a6} may be bonded to each other via a single bond or a divalent linking group to form a ring.

Examples of the divalent linking group include -O-, -S-, an alkylene group, a silylene group, an alkenylene group, a cycloalkylene group, a cycloalkenylene group, an arylene group, a divalent heterocyclic group, and an imino group.

Examples of the ring formed by bonding R^{a5} and R^{a6} to each other include an aromatic ring (an aromatic hydrocarbon ring or an aromatic heterocyclic ring) and a non-aromatic ring.

Examples of the aromatic ring include a benzene ring and a fluorene ring.

Furthermore, in a case where R^{a5} and R^{a6} are bonded to each other to form a ring, the compound represented by Formula (3) is preferably a compound represented by Formula (3A).

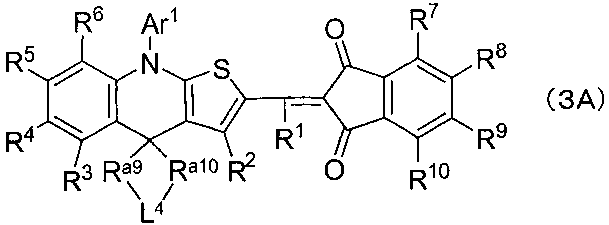

The definitions of Ar¹ and R¹ to R¹⁰ in Formula (3A) are the same as the definitions of Ar¹ and R¹ to R¹⁰ in Formula (3), and preferred aspects thereof are also the same.

R^{a9} and R^{a10} each independently represent an arylene group or a heteroarylene group.

The definition of the arylene group or heteroarylene group represented by R^{a9} and R^{a10} is the same as the definition of the arylene group or heteroarylene group represented by R^{a3} and R^{a4} in Formula (1A), and preferred aspects thereof are also the same.

L⁴ represents a single bond or a divalent linking group.

The definition of the divalent linking group represented by L⁴ is the same as the definition of the divalent linking group represented by L² in Formula (1A), and preferred aspects thereof are also the same.

R⁷ and R⁸, R⁸ and R⁹, and R⁹ and R¹⁰ may be each independently bonded to each other to form a ring. Examples of the type of the ring to be formed include an aromatic ring (an aromatic hydrocarbon ring or an aromatic heterocyclic ring) and a non-aromatic ring. Examples of the aromatic ring include a benzene ring and a fluorene ring.

In Formula (3) and Formula (3A), from the viewpoint that the effect of the present invention is superior, it is preferable that at least one of Ar¹, R³, ..., or R⁶ represents the group represented by Formula (4A), the group represented by Formula (4B), or the group represented by Formula (5), and more preferable that Ar¹ represents the group represented by Formula (4A), the group represented by Formula (4B), or the group represented by Formula (5) and at least one of R³, ..., or R⁶ represents the group represented by Formula (4A) or the group represented by Formula (4B).

From the viewpoint that the effect of the present invention is superior, the specific compound preferably corresponds to any of the following [1] or [2].
[1] The specific compound is a compound represented by Formula (3), and in the compound, Ar¹ represents the group represented by Formula (4A), the group represented by Formula (4B), or the group represented by Formula (5) and at least one of R³, ..., or R⁶ represents the group represented by Formula (4A) or the group represented by Formula (4B).
[2] The specific compound is a compound represented by Formula (3A).

### (Substituent W)

The substituent W in the present specification will be described.

Examples of the substituent W include a halogen atom (a fluorine atom, a chlorine atom, a bromine atom, an iodine atom, and the like), an alkyl group (including a cycloalkyl group, a bicycloalkyl group, and a tricycloalkyl group), an alkenyl group (including a cycloalkenyl group and a bicycloalkenyl group), an alkynyl group, an aryl group, a heterocyclic group (may be referred to as a heterocycle group and includes a heteroaryl group), a cyano group, a hydroxy group, a nitro group, an alkoxy group, an aryloxy group, a silyloxy group, a heterocyclic oxy group, an acyloxy group, a carbamoyloxy group, an alkoxycarbonyloxy group, an aryloxycarbonyloxy group, an amino group (including an anilino group), an ammonio group, an acylamino group, an aminocarbonylamino group, an alkoxycarbonylamino group, an aryloxycarbonylamino group, a sulfamoylamino group, an alkyl- or aryl-sulfonylamino group, a mercapto group, an alkylthio group, an arylthio group, a heterocyclic thio group, a sulfamoyl group, an alkyl- or aryl-sulfinyl group, an alkyl- or aryl-sulfonyl group, an acyl group, an aryloxycarbonyl group, an alkoxycarbonyl group, a carbamoyl group, an aryl- or heterocyclic azo group, an imide group, a phosphino group, a phosphinyl group, a phosphinyloxy group, a phosphinylamino group, a phosphono group, a silyl group, a hydrazino group, a ureido group, and a boronic acid group (-B(OH)₂).

Furthermore, the substituent W may be further substituted with the substituent W. For example, an alkyl group may be substituted with a halogen atom.

The specific compounds are exemplified below, but the specific compound according to the embodiment of the present invention is not limited thereto.

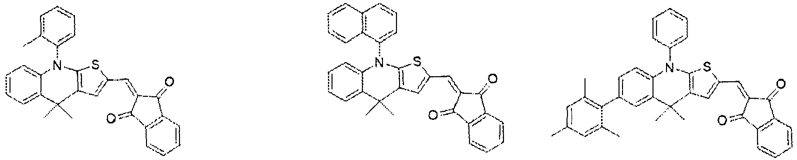

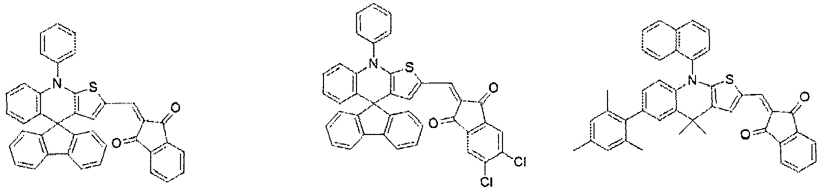

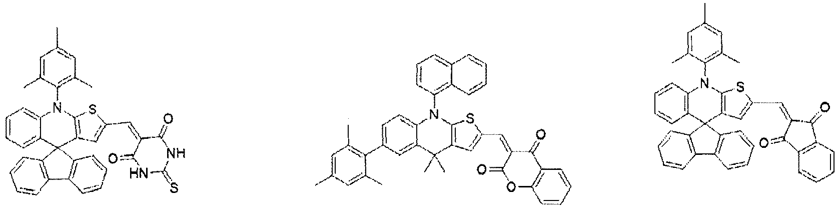

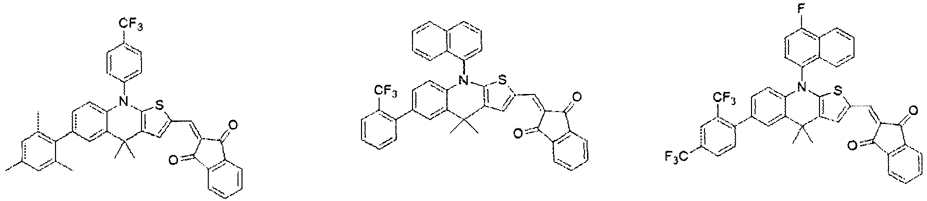

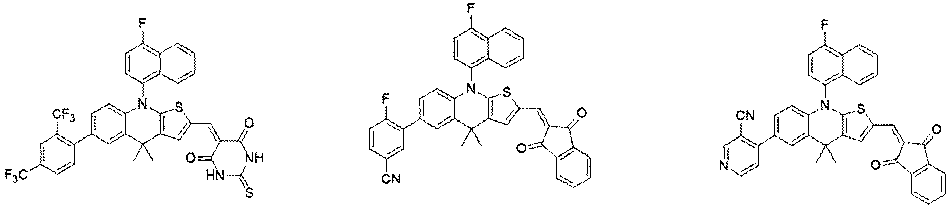

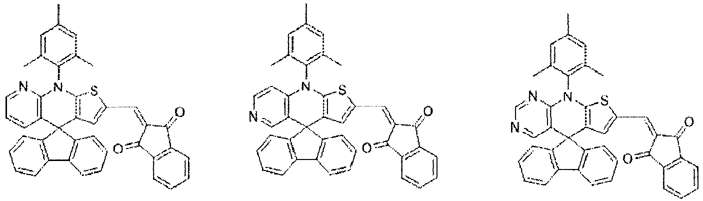

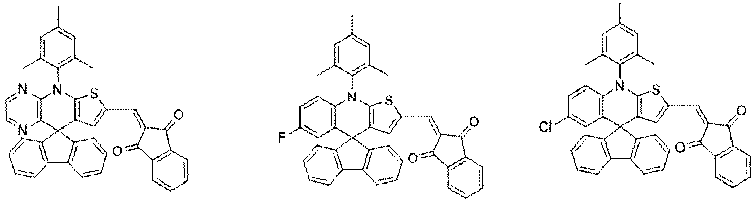

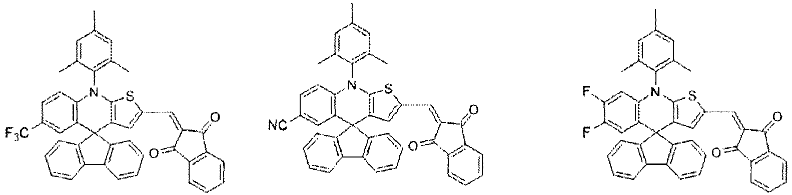

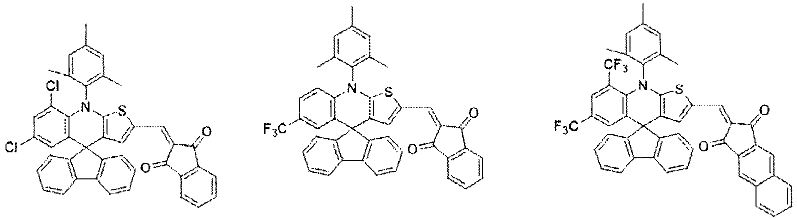

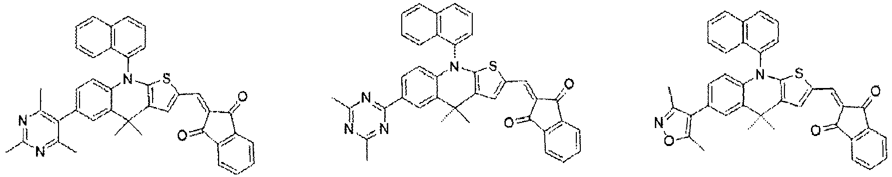

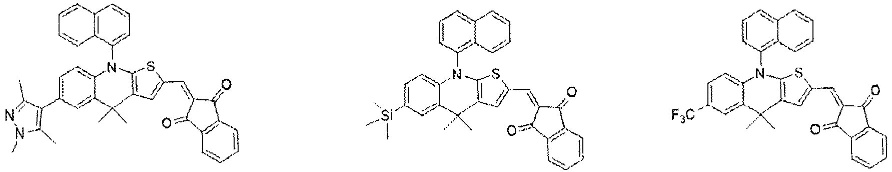

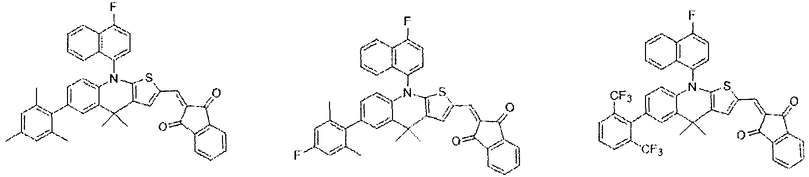

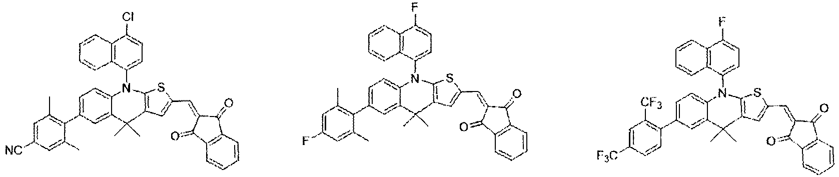

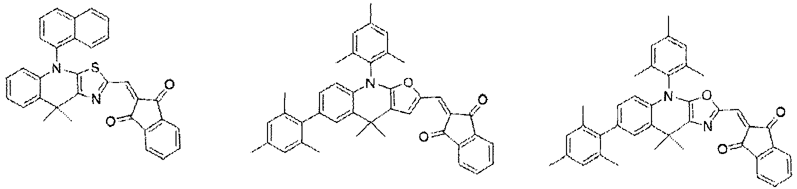

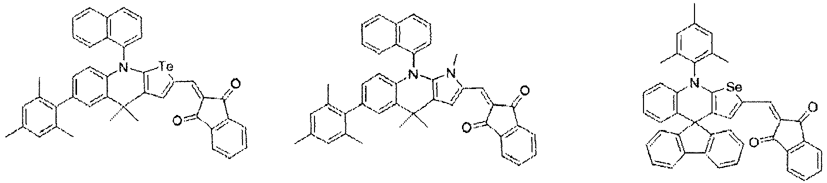

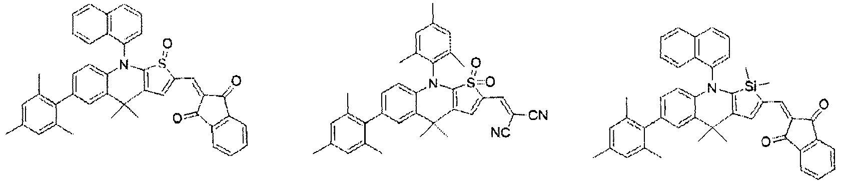

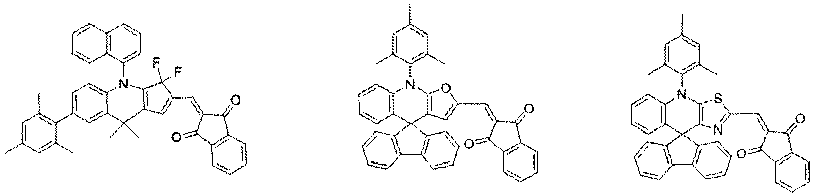

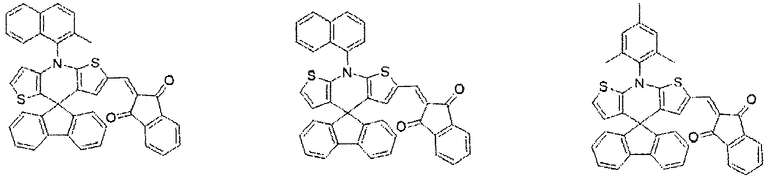

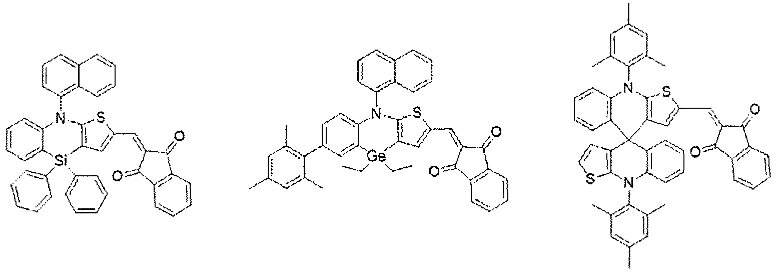

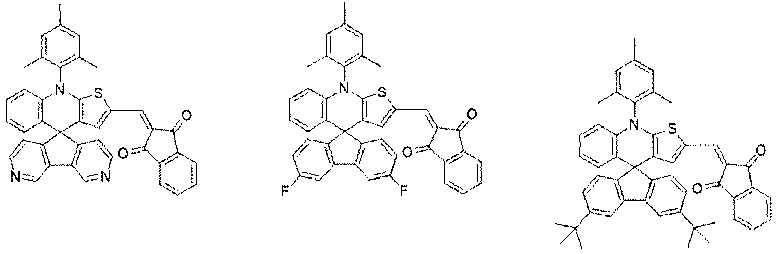

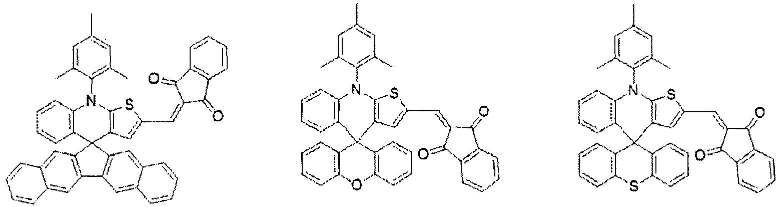

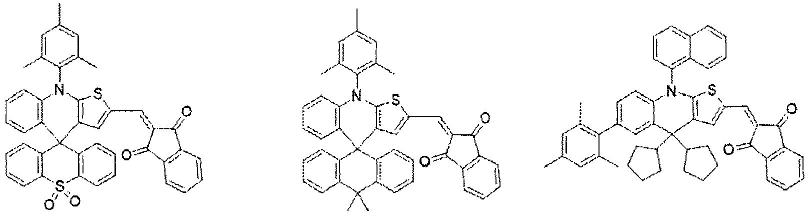

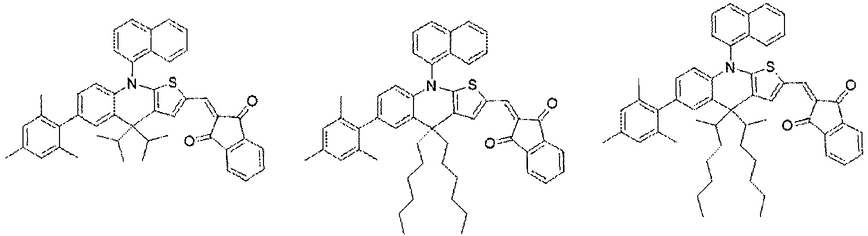

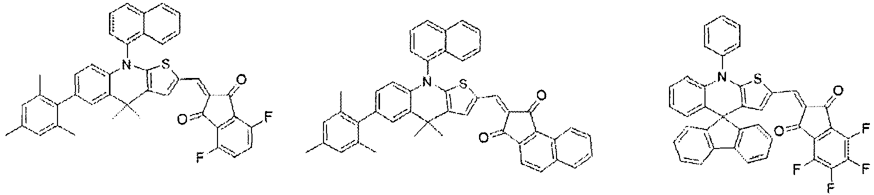

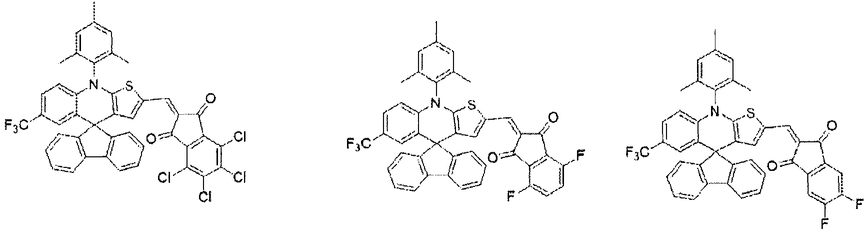

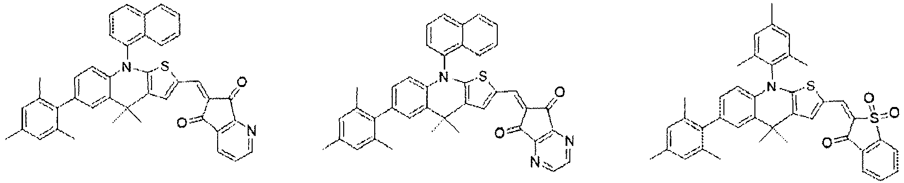

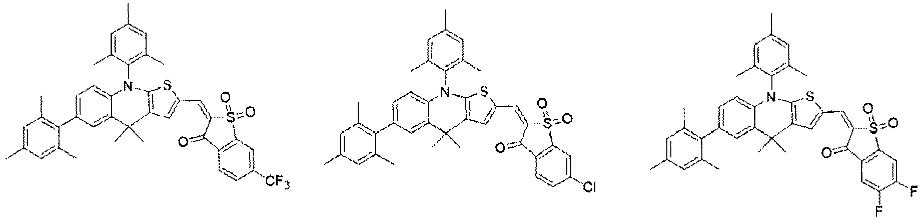

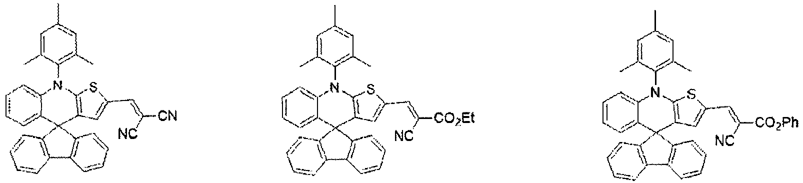

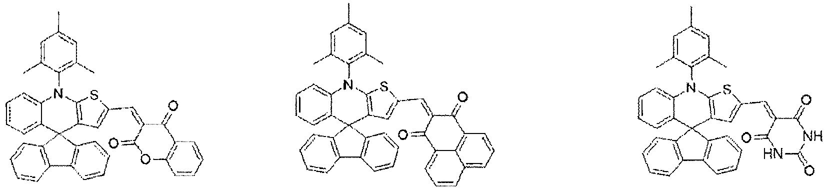

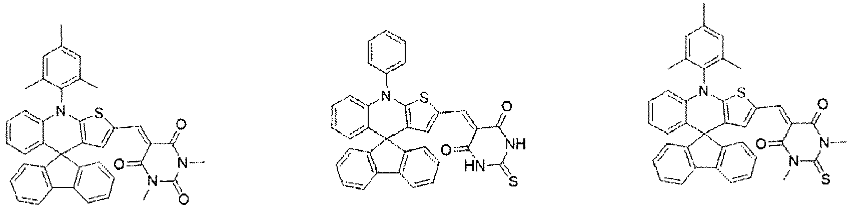

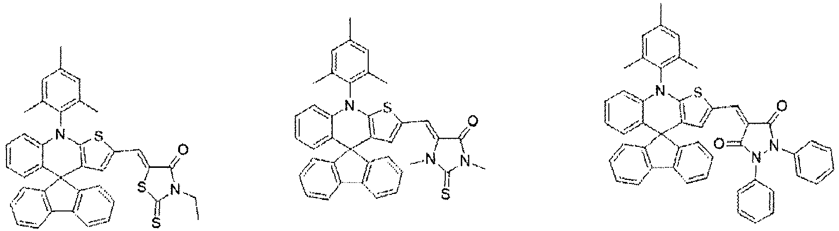

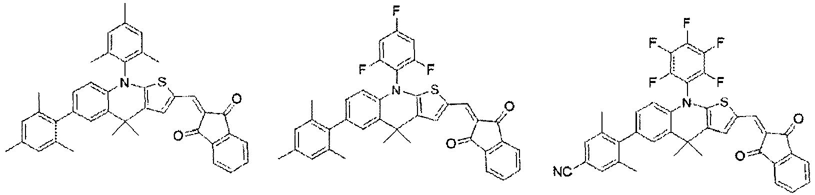

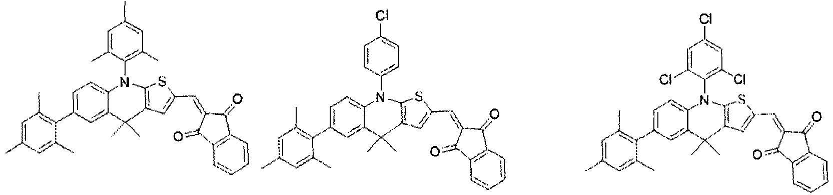

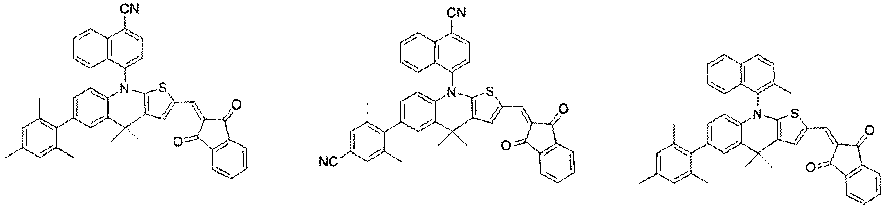

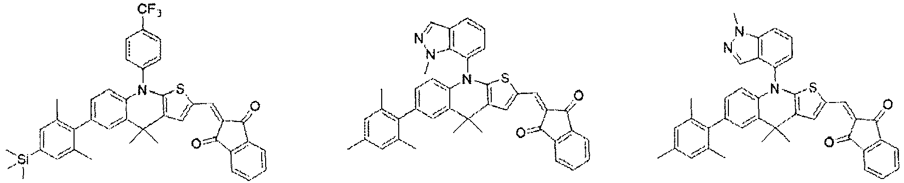

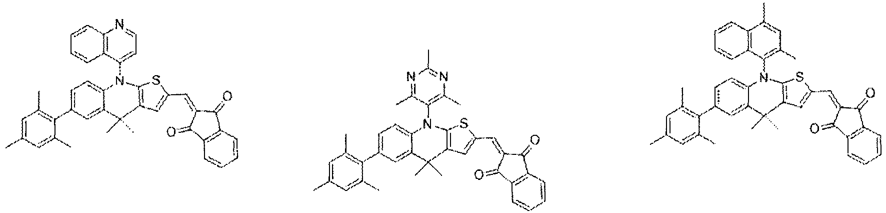

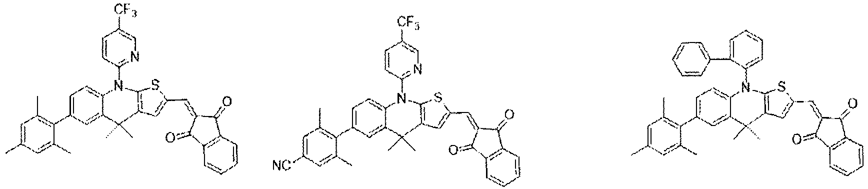

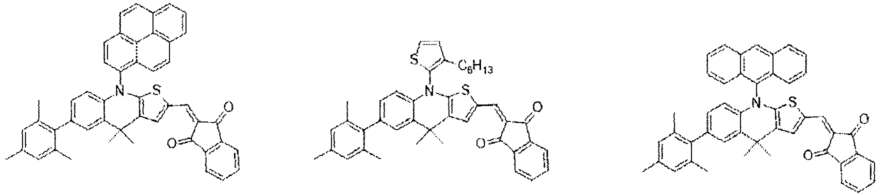

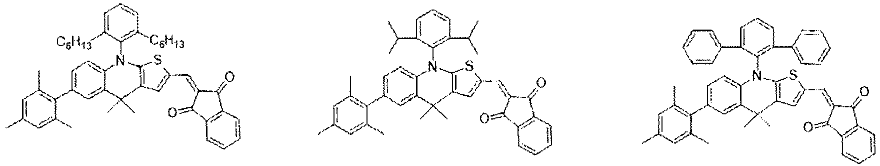

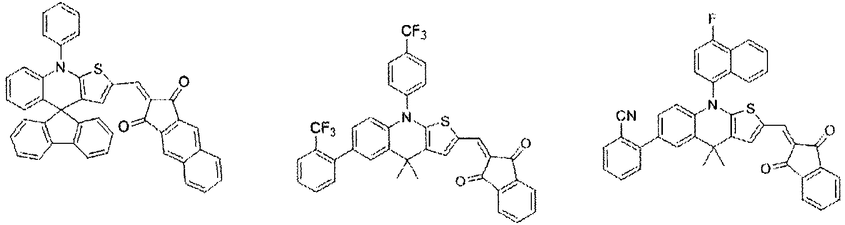

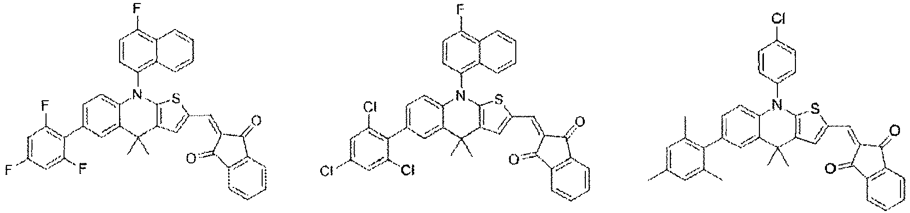

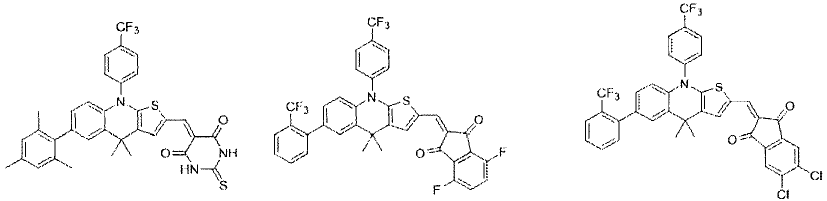

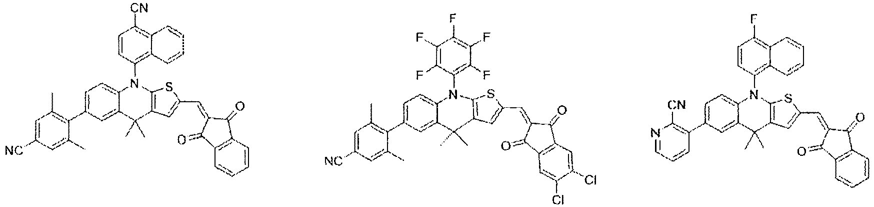

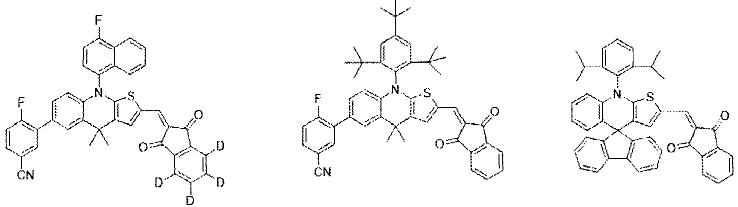

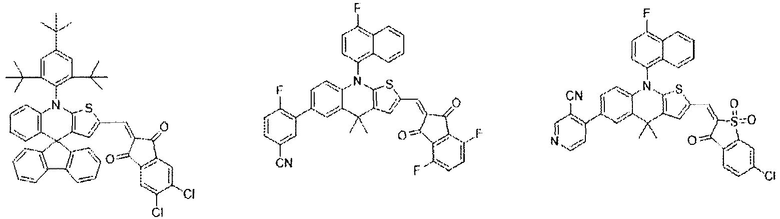

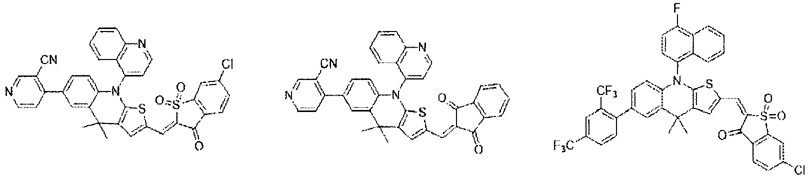

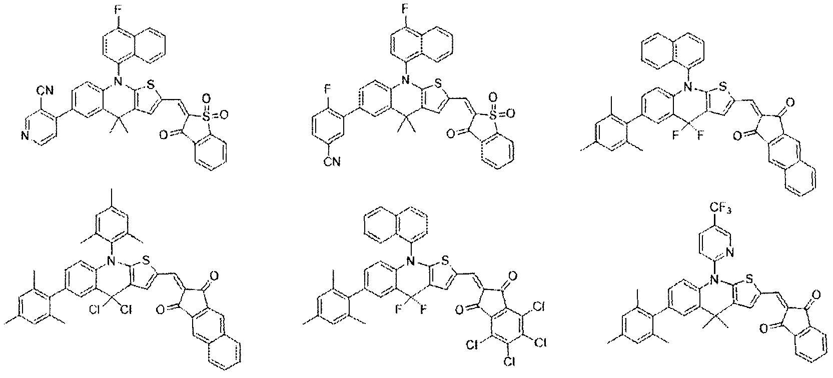

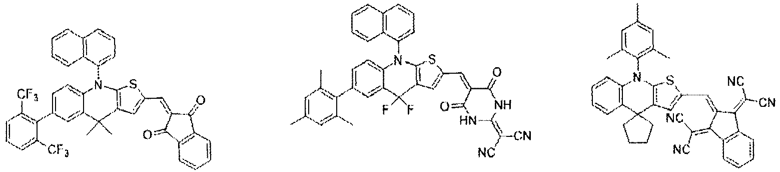

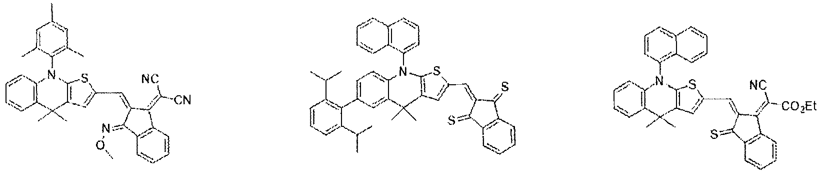

The specific compound is particularly useful as a material of the photoelectric conversion film used for an imaging element, an optical sensor, or a photoelectric cell. Moreover, in general, the specific compound functions as a p-type organic semiconductor in the photoelectric conversion film in many cases. Furthermore, the specific compound can also be used as a coloring material, a liquid crystal material, an organic semiconductor material, a charge transport material, a pharmaceutical material, and a fluorescent diagnostic agent material.

The specific compound is preferably a compound in which an ionization potential in a single film is -5.0 to -6.0 eV from the viewpoints of stability in a case of using the compound as the p-type organic semiconductor and matching of energy levels between the compound and an n-type organic semiconductor.

The maximum absorption wavelength of the specific compound is not particularly limited, but is preferably in a range of 510 to 570 nm and more preferably in a range of 520 to 560 nm from the viewpoint that the photoelectric conversion film in the photoelectric conversion element according to the embodiment of the present invention is suitably used as an organic photoelectric conversion film which receives (absorbs) green light and performs photoelectric conversion.

The absorption half-width of the specific compound is not particularly limited, but is preferably less than 100 nm, more preferably less than 95 nm, and still more preferably less than 90 nm from the viewpoint that the photoelectric conversion film in the photoelectric conversion element according to the embodiment of the present invention is suitably used as an organic photoelectric conversion film which receives (absorbs) green light and performs photoelectric conversion. The lower limit thereof is not particularly limited, but is 60 nm or greater in many cases.

Furthermore, the maximum absorption wavelength and the absorption half-width are values measured in a state where the specific compound is in a form of a film (for example, a vapor-deposited film of the specific compound).

The maximum absorption wavelength of the photoelectric conversion film is not particularly limited, but is preferably in a range of 510 to 570 nm and more preferably in a range of 520 to 560 nm from the viewpoint that the photoelectric conversion film in the photoelectric conversion element according to the embodiment of the present invention is suitably used as an organic photoelectric conversion film which receives (absorbs) green light and performs photoelectric conversion.

### <n-type organic semiconductor>

The photoelectric conversion film preferably contains the n-type organic semiconductor as a component other than the specific compound.

The n-type organic semiconductor is an acceptor-property organic semiconductor material (a compound), and refers to an organic compound having a property of easily accepting an electron. More specifically, the n-type organic semiconductor refers to an organic compound having a larger electron affinity in a case where two organic compounds are used in contact with each other. Therefore, any organic compound can be used as an acceptor-property organic semiconductor as long as the organic compound has an electron accepting property.

Examples of the n-type organic semiconductor include fullerenes selected from the group consisting of a fullerene and a derivative thereof, a fused aromatic carbocyclic compound (for example, a naphthalene derivative, an anthracene derivative, a phenanthrene derivative, a tetracene derivative, a pyrene derivative, a perylene derivative, and a fluoranthene derivative); a 5- to 7-membered heterocyclic compound having at least one of a nitrogen atom, an oxygen atom, or a sulfur atom (for example, pyridine, pyrazine, pyrimidine, pyridazine, triazine, quinoline, quinoxaline, quinazoline, phthalazine, cinnoline, isoquinoline, pteridine, acridine, phenazine, phenanthroline, tetrazole, pyrazole, imidazole, thiazole, and the like); a polyarylene compound; a fluorene compound; a cyclopentadiene compound; a silyl compound; a 1,4,5,8-naphthalenetetracarboxylic acid anhydride; a 1,4,5,8-naphthalenetetracarboxylic acid anhydride imide derivative; an oxadiazole derivative; an anthraquinodimethane derivative; a diphenylquinone derivative; bathocuproine, bathophenanthroline, and derivatives thereof; triazole compounds; a distyrylarylene derivative; a metal complex having a nitrogen-containing heterocyclic compound as a ligand; a silole compound; and the compounds described in paragraphs [0056] and [0057] of JP2006-100767A.

Among them, as the n-type organic semiconductor (the compound), an n-type organic semiconductor containing fullerenes selected from the group consisting of a fullerene and a derivative thereof is preferable.

Examples of fullerene include fullerene C60, fullerene C70, fullerene C76, fullerene C78, fullerene C80, fullerene C82, fullerene C84, fullerene C90, fullerene C96, fullerene C240, fullerene C540, and a mixed fullerene.

Examples of fullerene derivative include compounds in which a substituent is added to the above-described fullerenes. As the substituent, an alkyl group, an aryl group, or a heterocyclic group is preferable. As the fullerene derivative, the compounds described in JP2007-123707A are preferable.

In addition, an organic dye may be used as the n-type organic semiconductor. Examples of the organic dye include a cyanine dye, a styryl dye, a hemicyanine dye, a merocyanine dye (including zeromethine merocyanine (simple merocyanine)), a rhodacyanine dye, an allopolar dye, an oxonol dye, a hemioxonol dye, a squarylium dye, a croconium dye, an azamethine dye, a coumarin dye, an arylidene dye, an anthraquinone dye, a triphenylmethane dye, an azo dye, an azomethine dye, a metallocene dye, a fluorenone dye, a fulgide dye, a perylene dye, a phenazine dye, a phenothiazine dye, a quinone dye, a diphenylmethane dye, a polyene dye, an acridine dye, an acridinone dye, a diphenylamine dye, a quinophthalone dye, a phenoxazine dye, a phthaloperylene dye, a dioxane dye, a porphyrin dye, a chlorophyll dye, a phthalocyanine dye, a subphthalocyanine dye, and a metal complex dye.

The molecular weight of the n-type organic semiconductor is preferably 200 to 1200 and more preferably 200 to 900.

From the viewpoint that the photoelectric conversion film in the photoelectric conversion element according to the embodiment of the present invention is suitably used as an organic photoelectric conversion film which receives (absorbs) green light and performs photoelectric conversion, it is desirable that the n-type organic semiconductor is colorless or has an absorption maximum wavelength and/or an absorption waveform close to that of the specific compound, and as the specific numerical value, the absorption maximum wavelength of the n-type organic semiconductor is preferably 400 nm or less or in the range of 500 to 600 nm.

The photoelectric conversion film preferably has a bulk hetero structure formed in a state in which the specific compound and the n-type organic semiconductor are mixed. The bulk hetero structure refers to a layer in which the specific compound and the n-type organic semiconductor are mixed and dispersed in the photoelectric conversion film. The photoelectric conversion film having the bulk hetero structure can be formed by either a wet method or a dry method. Moreover, the bulk hetero structure is described in detail in paragraphs [0013] and [0014] of JP2005-303266A.

From the viewpoint of responsiveness of the photoelectric conversion element, the content of the specific compound to the total content of the specific compound and the n-type organic semiconductor (= film thickness in terms of single layer of specific compound/(film thickness in terms of single layer of specific compound + film thickness in terms of single layer of n-type organic semiconductor) × 100) is preferably 20 to 80 volume% and more preferably 40 to 80 volume%.

Furthermore, it is preferable that the photoelectric conversion film is substantially formed of the specific compound and the n-type organic semiconductor. The term "substantially" means that the total content of the specific compound and the n-type organic semiconductor with respect to the total mass of the photoelectric conversion film is 95 mass% or greater.

In addition, the n-type organic semiconductor contained in the photoelectric conversion film may be used alone or in combination of two or more thereof.

In addition to the specific compound and the n-type organic semiconductor, the photoelectric conversion film may further contain the p-type organic semiconductor. Examples of the p-type organic semiconductor include the compounds shown below.

The p-type organic semiconductor here means a p-type organic semiconductor which is a compound different from the specific compound. Moreover, in a case where the photoelectric conversion film contains the p-type organic semiconductor, the p-type organic semiconductor may be used alone or in combination of two or more thereof.

### <p-type organic semiconductor>

The p-type organic semiconductor is a donor-property organic semiconductor material (a compound), and refers to an organic compound having a property of easily donating an electron. More specifically, the p-type organic semiconductor refers to an organic compound having a smaller ionization potential in a case where two organic compounds are used in contact with each other.

Examples of the p-type organic semiconductor include a triarylamine compound (for example, N,N'-bis(3-methylphenyl)-(1,1'-biphenyl)-4,4'-diamine (TPD), 4,4'-bis[N-(naphthyl)-N-phenyl-amino] biphenyl (a-NPD), the compounds described in paragraphs [0128] to [0148] of JP2011-228614A, the compounds described in paragraphs [0052] to [0063] of JP2011-176259A, the compounds described in paragraphs [0119] to [0158] of JP2011-225544A, the compounds described in [0044] to [0051] of JP2015-153910A, the compounds described in paragraphs [0086] to [0090] of JP2012-094660A, and the like), a pyrazoline compound, a styrylamine compound, a hydrazone compound, a polysilane compound, a thiophene compound (for example, a thienothiophene derivative, a dibenzothiophene derivative, a benzodithiophene derivative, a dithienothiophene derivative, a [1]benzothieno[3,2-b]thiophene (BTBT) derivative, a thieno[3,2-f:4,5-f']bis[1]benzothiophene (TBBT) derivative, the compounds described in paragraphs [0031] to [0036] of JP2018-014474A, the compounds described in paragraphs [0043] to [0045] of WO2016-194630A, the compounds described in paragraphs [0025] to [0037] and [0099] to [0109] of WO2017-159684A, and the like), a cyanine compound, an oxonol compound, a polyamine compound, an indole compound, a pyrrole compound, a pyrazole compound, a polyarylene compound, a fused aromatic carbocyclic compound (for example, a naphthalene derivative, an anthracene derivative, a phenanthrene derivative, a tetracene derivative, a pentacene derivative, a pyrene derivative, a perylene derivative, and a fluoranthene derivative), a porphyrin compound, a phthalocyanine compound, a triazole compound, an oxadiazole compound, an imidazole compound, a polyarylalkane compound, a pyrazolone compound, an amino-substituted chalcone compound, an oxazole compound, a fluorenone compound, a silazane compound, and a metal complex having a nitrogen-containing heterocyclic compound as a ligand.

Examples of the p-type organic semiconductor include compounds having an ionization potential smaller than that of the n-type organic semiconductor, and in a case where this condition is satisfied, the organic dyes exemplified as the n-type organic semiconductor can be used.

The compounds which can be used as the p-type semiconductor compound are exemplified below.

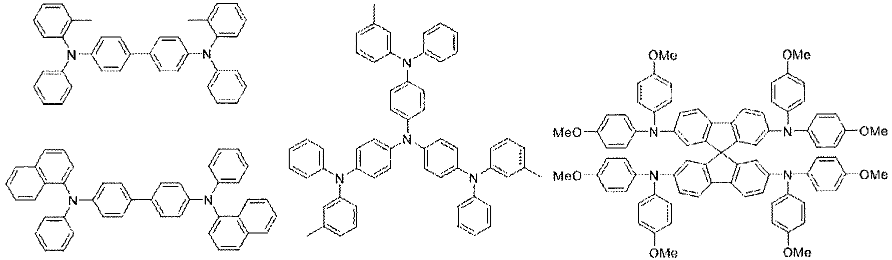

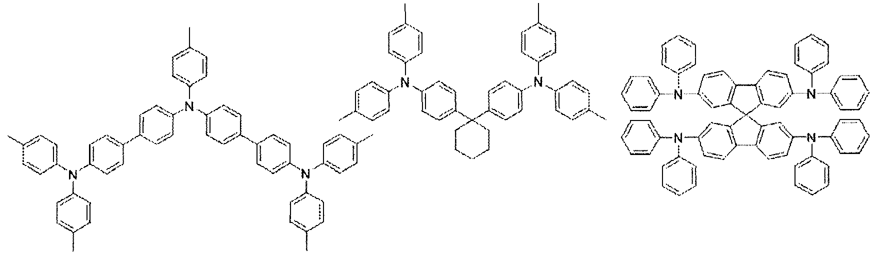

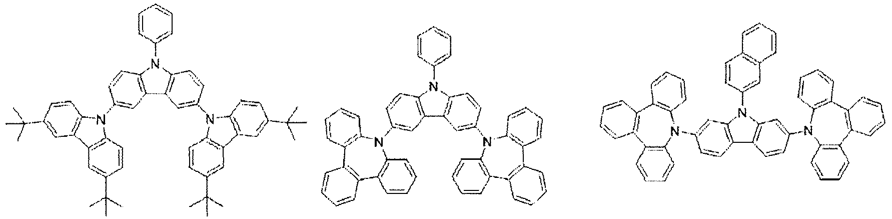

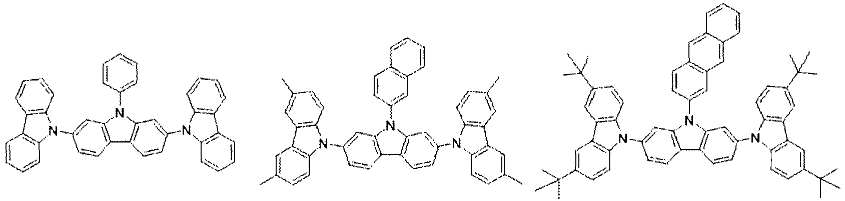

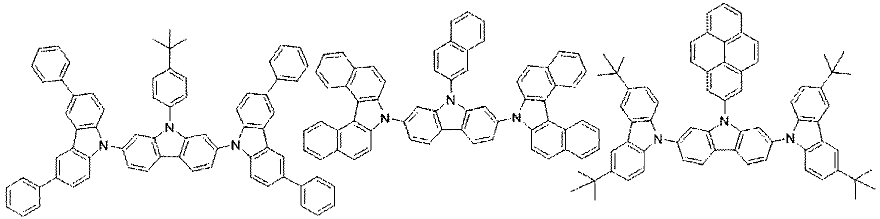

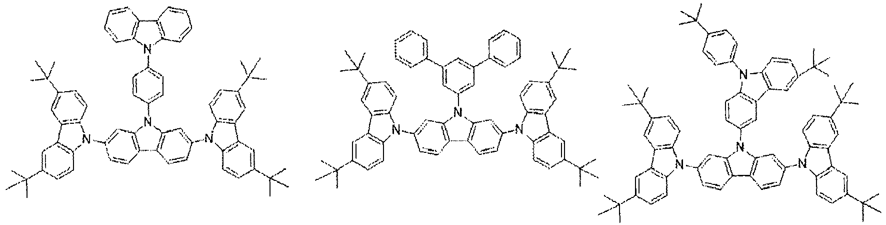

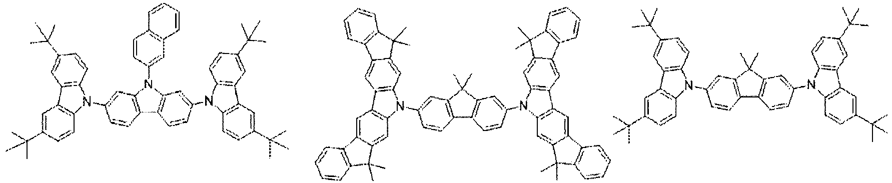

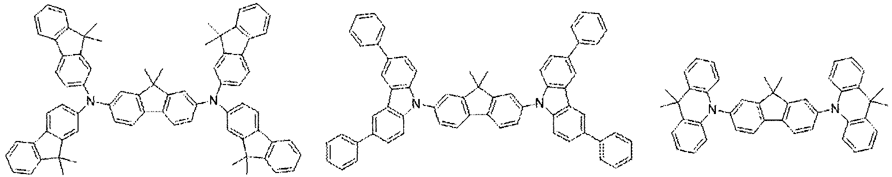

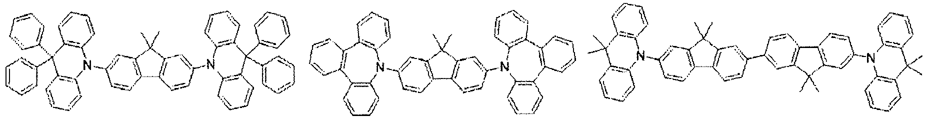

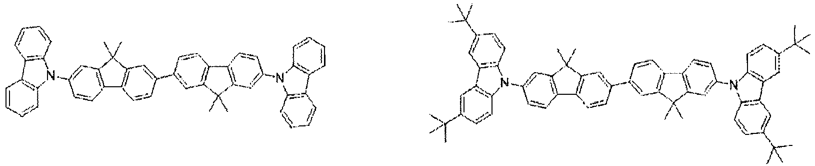

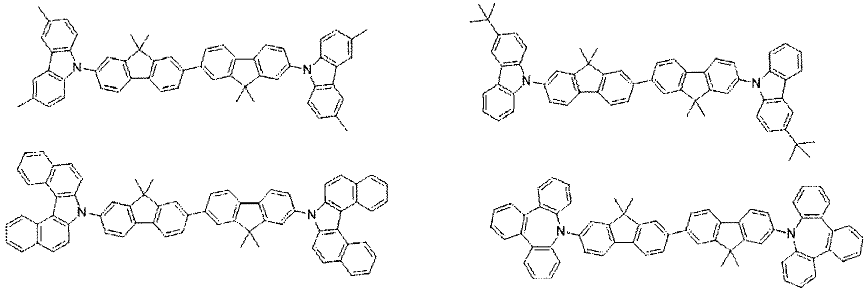

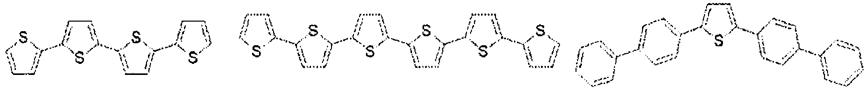

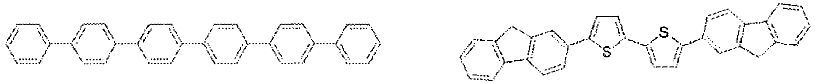

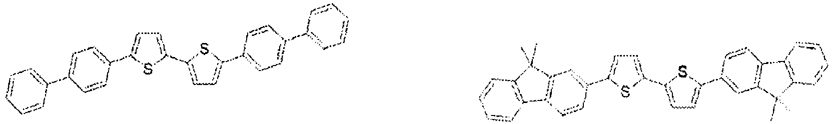

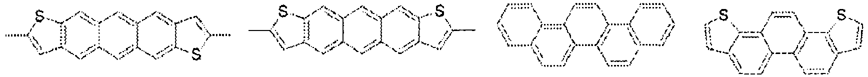

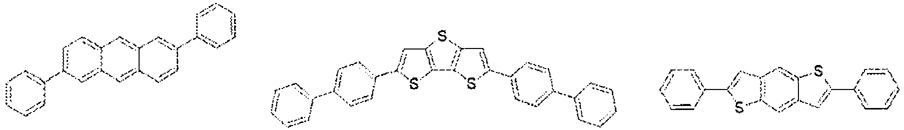

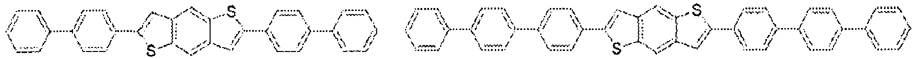

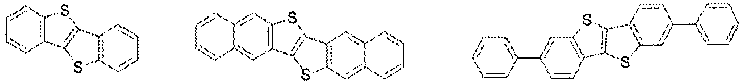

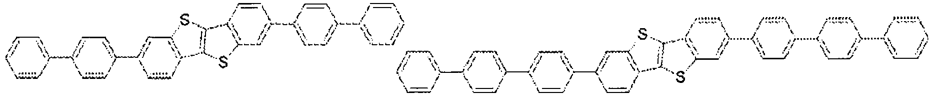

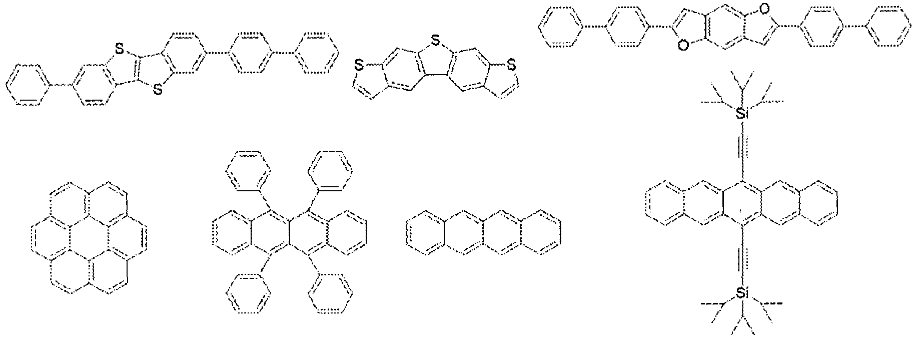

The photoelectric conversion film containing the specific compound is a non-luminescent film, and has features different from that of an organic light emitting diode (OLED). The non-luminescent film means a film having a luminescence quantum efficiency of 1% or less, and the luminescence quantum efficiency is preferably 0.5% or less and more preferably 0.1% or less.

### <Film Formation Method>

The photoelectric conversion film can be formed mostly by a dry film formation method. Examples of the dry film formation method include a physical vapor-phase growth method such as a vapor deposition method (in particular, a vacuum deposition method), a sputtering method, an ion plating method, and a molecular beam epitaxy (MBE) method, and a chemical vapor deposition (CVD) method such as plasma polymerization. Among them, the vacuum deposition method is preferable. In a case where the photoelectric conversion film is formed by the vacuum deposition method, production conditions such as a degree of vacuum and a vapor deposition temperature can be set according to the normal method.

The thickness of the photoelectric conversion film is preferably 10 to 1000 nm, more preferably 50 to 800 nm, still more preferably 50 to 500 nm, and particularly preferably 50 to 300 nm.

### <Electrode>

The electrode (the upper electrode (the transparent conductive film) 15 and the lower electrode (the conductive film) 11) is formed of a conductive material. Examples of the conductive material include a metal, an alloy, a metal oxide, an electrically conductive compound, and a mixture thereof.

Since light is incident through the upper electrode 15, the upper electrode 15 is preferably transparent to light to be detected. Examples of the material constituting the upper electrode 15 include a conductive metal oxide such as tin oxide (antimony tin oxide (ATO) or fluorine doped tin oxide (FTO)) doped with antimony, fluorine, or the like, tin oxide, zinc oxide, indium oxide, indium tin oxide (ITO), and indium zinc oxide (IZO); a metal thin film such as gold, silver, chromium, and nickel; a mixture or laminate of these metals and the conductive metal oxide; and an organic conductive material such as polyaniline, polythiophene, and polypyrrole. Among them, a conductive metal oxide is preferable from the viewpoints of high conductivity, transparency, and the like.

In general, in a case where the conductive film is made to be thinner than a certain range, a resistance value is rapidly increased. However, in the solid-state imaging element into which the photoelectric conversion element according to the present embodiment is incorporated, the sheet resistance is preferably 100 to 10000 Ω/□, and the degree of freedom of the range of the film thickness which can be thinned is large. In addition, as the thickness of the upper electrode (the transparent conductive film) 15 is thinner, the amount of light absorbed by the upper electrode is smaller, and the light transmittance usually is increased. The increase in the light transmittance causes an increase in light absorption in the photoelectric conversion film and an increase in the photoelectric conversion ability, which is preferable. Considering the suppression of leakage current, an increase in the resistance value of the thin film, and an increase in transmittance accompanied by the thinning, the film thickness of the upper electrode 15 is preferably 5 to 100 nm and more preferably 5 to 20 nm.

There is a case where the lower electrode 11 has transparency and, on the contrary, a case where the lower electrode does not have transparency and reflects light, depending on the application. Examples of a material constituting the lower electrode 11 include a conductive metal oxide such as tin oxide (ATO, FTO) doped with antimony, fluorine, or the like, tin oxide, zinc oxide, indium oxide, indium tin oxide (ITO), and indium zinc oxide (IZO); a metal such as gold, silver, chromium, nickel, titanium, tungsten, and aluminum, a conductive compound (for example, titanium nitride (TiN)) such as oxides or nitrides of these metals; a mixture or laminate of these metals and the conductive metal oxide; and an organic conductive material such as polyaniline, polythiophene, and polypyrrole.

The method of forming electrodes is not particularly limited, and can be appropriately selected in accordance with the electrode material. Specific examples thereof include a wet method such as a printing method and a coating method; a physical method such as a vacuum deposition method, a sputtering method, and an ion plating method; and a chemical method such as CVD and a plasma CVD method.

In a case where the material of the electrode is ITO, examples of the method include an electron beam method, a sputtering method, a resistance heating vapor deposition method, a chemical reaction method (such as a sol-gel method), and coating with a dispersion of indium tin oxide.

### <Charge blocking film: electron blocking film and positive hole blocking film>

The photoelectric conversion element according to the embodiment of the present invention preferably has one or more interlayers between the conductive film and the transparent conductive film, in addition to the photoelectric conversion film. Examples of the interlayer include a charge blocking film. In the case where the photoelectric conversion element has this film, the characteristics (such as photoelectric conversion efficiency, responsiveness, and the like) of the photoelectric conversion element to be obtained are superior. Examples of the charge blocking film include the electron blocking film and the positive hole blocking film. Hereinafter, the respective films will be described in detail.

### (Electron blocking film)

The electron blocking film is a donor-property organic semiconductor material (a compound), and the p-type organic semiconductor described above can be used.

Moreover, a polymer material can also be used as the electron blocking film.

Examples of the polymer material include a polymer such as phenylenevinylene, fluorene, carbazole, indole, pyrene, pyrrole, picoline, thiophene, acetylene, and diacetylene, and a derivative thereof.

### In addition, the electron blocking film may be formed with a plurality of films.

The electron blocking film may be formed of an inorganic material. In general, the inorganic material has a dielectric constant larger than that of an organic material. Therefore, in a case where the inorganic material is used for the electron blocking film, a large voltage is applied to the photoelectric conversion film, and thus the photoelectric conversion efficiency is increased. Examples of the inorganic material which can be used for the electron blocking film include calcium oxide, chromium oxide, chromium copper oxide, manganese oxide, cobalt oxide, nickel oxide, copper oxide, gallium copper oxide, copper strontium oxide, niobium oxide, molybdenum oxide, indium copper oxide, indium silver oxide, and iridium oxide.

### (Positive hole blocking film)

A positive hole blocking film is an acceptor-property organic semiconductor material (a compound), and the n-type semiconductor described above can be used.

The method of producing the charge blocking film is not particularly limited, but a dry film formation method and a wet film formation method are exemplified. Examples of the dry film formation method include a vapor deposition method and a sputtering method. The vapor deposition method may be any of a physical vapor deposition (PVD) method or a chemical vapor deposition (CVD) method, and the physical vapor deposition method such as a vacuum deposition method is preferable. Examples of the wet film formation method include an ink jet method, a spray method, a nozzle printing method, a spin coating method, a dip coating method, a casting method, a die coating method, a roll coating method, a bar coating method, and a gravure coating method, and from the viewpoint of high precision patterning, an ink jet method is preferable.

Each thickness of the charge blocking films (the electron blocking film and the positive hole blocking film) is preferably 3 to 200 nm, more preferably 5 to 100 nm, and still more preferably 5 to 30 nm.

### <Substrate>

The photoelectric conversion element may further include a substrate. The type of substrate to be used is not particularly limited, but a semiconductor substrate, a glass substrate, and a plastic substrate are exemplified.

Furthermore, the position of the substrate is not particularly limited, but in general, the conductive film, the photoelectric conversion film, and the transparent conductive film are laminated in this order on the substrate.

### <Sealing layer>

The photoelectric conversion element may further include a sealing layer. The performance of the photoelectric conversion material may significantly deteriorate due to the presence of a deterioration factor such as a water molecule. Accordingly, the deterioration can be prevented by coating and sealing the entire photoelectric conversion film with a sealing layer such as diamond-like carbon (DLC) or ceramics such as a metal oxide, a metal nitride, or a metal nitride oxide which are dense and do not allow a water molecule to permeate.

Furthermore, regarding the sealing layer, the selection of the material and the production may be performed according to the description in paragraphs [0210] to [0215] of JP2011-082508A.

### [Imaging element]

Examples of the application of the photoelectric conversion element include an imaging element. The imaging element is an element which converts optical information of an image into an electric signal, and, in general, refers to an element in which a plurality of photoelectric conversion elements are arranged in a matrix on the same plane, an optical signal is converted into an electric signal in each photoelectric conversion element (a pixel), and the electric signal can be sequentially output to the outside of the imaging element for each pixel. Therefore, each pixel includes one or more photoelectric conversion elements and one or more transistors.

Fig. 3 is a schematic cross-sectional view showing a schematic configuration of an imaging element for describing one embodiment of the present invention. This imaging element is mounted on an imaging element such as a digital camera and a digital video camera, an electronic endoscope, and an imaging module such as a cellular phone.

An imaging element 20a shown in Fig. 3 includes a photoelectric conversion element 10a according to the embodiment of the present invention, a blue photoelectric conversion element 22, and a red photoelectric conversion element 24, which are laminated along the light incident direction. As described above, the photoelectric conversion element 10a can mainly function as a green photoelectric conversion element capable of receiving green light.

The imaging element 20a is a so-called laminate-type color separation imaging element. The photoelectric conversion element 10a, the blue photoelectric conversion element 22, and the red photoelectric conversion element 24 have different wavelength spectra to be detected. That is, the blue photoelectric conversion element 22 and the red photoelectric conversion element 24 correspond to photoelectric conversion elements which receive (absorb) light having a wavelength different from a wavelength of the light received by the photoelectric conversion element 10a. The photoelectric conversion element 10a can mainly receive green light, the blue photoelectric conversion element 22 can mainly receive blue light, and the red photoelectric conversion element can mainly receive red light.

In addition, the green light means light in the wavelength range of 500 to 600 nm, the blue light means light in the wavelength range of 400 to 500 nm, and the red light means light in the wavelength range of 600 to 700 nm.

In a case where light is incident on the imaging element 20a in the direction of the arrow, first, green light is mainly absorbed by the photoelectric conversion element 10a, but blue light and red light are transmitted through the photoelectric conversion element 10a. In a case where the light transmitted through the photoelectric conversion element 10a travels to the blue photoelectric conversion element 22, the blue light is mainly absorbed, but the red light is transmitted through the blue photoelectric conversion element 22. Thereafter, light transmitted through the blue photoelectric conversion element 22 is absorbed by the red photoelectric conversion element 24. As described above, in the imaging element 20a, which is a lamination-type color separation imaging element, one pixel can be configured with three light receiving sections of green, blue, and red, and the area of each light receiving section can be made large.

In particular, as described above, the photoelectric conversion element 10a according to the embodiment of the present invention has a narrow half-width of an absorption peak, and thus absorption of blue light and red light hardly occurs, and the detectability of the blue photoelectric conversion element 22 and the red photoelectric conversion element 24 is less likely to be affected.

The configurations of the blue photoelectric conversion element 22 and the red photoelectric conversion element 24 are not particularly limited.

For example, the photoelectric conversion element having a configuration in which colors are separated based on a difference in light absorption length by using silicon may be used. As a more specific example, both the blue photoelectric conversion element 22 and the red photoelectric conversion element 24 may be made of silicon. In this case, regarding the light consisting of the blue light, the green light, and the red light which are incident on the imaging element 20a in the direction of the arrow, the photoelectric conversion element 10a mainly receives the green light, which is light having the center wavelength, and the remaining blue light and red light are easily separated. The blue light and the red light have different light absorption lengths for silicon (wavelength dependence of an absorption coefficient for silicon), the blue light is easily absorbed near the surface of silicon, and the red light can penetrate to a relatively deep position in the silicon. Based on such a difference in light absorption length, blue light is mainly received by the blue photoelectric conversion element 22 existing at a shallower position, and red light is mainly received by the red photoelectric conversion element 24 existing at a deeper position.

Furthermore, the blue photoelectric conversion element 22 and the red photoelectric conversion element 24 may be the photoelectric conversion element (the blue photoelectric conversion element 22 or the red photoelectric conversion element 24) having a configuration in which a conductive film, an organic photoelectric conversion film having absorption maximum for blue light or red light, and the transparent conductive film are included in this order.

In Fig. 3, the photoelectric conversion element according to the embodiment of the present invention, the blue photoelectric conversion element, and the red photoelectric conversion element are arranged in this order from the light incident side, but the arrangement is not limited to the aspect, and other arrangement orders may be adopted. For example, the blue photoelectric conversion element, the photoelectric conversion element according to the embodiment of the present invention, and the red photoelectric conversion element may be arranged in this order from the light incident side.

As described above, the configuration in which the photoelectric conversion elements of the three primary colors of blue, green, and red are laminated has been described as the imaging element, but the two layers (two colors) or four layers (four colors) or more may be adopted.

For example, an aspect in which the photoelectric conversion element 10a according to the embodiment of the present invention may be disposed on the arranged blue photoelectric conversion element 22 and red photoelectric conversion element 24 may be adopted. Furthermore, as needed, a color filter which absorbs light having a predetermined wavelength may be further disposed on the light incident side.

The form of the imaging element is not limited to the form shown in Fig. 3 and the form described above, and may be other forms.

For example, an aspect in which the photoelectric conversion element according to the embodiment of the present invention, the blue photoelectric conversion element, and the red photoelectric conversion element are arranged at a position in the same plane may be adopted.

In addition, the photoelectric conversion element may be used as a single layer. For example, blue, red, and green color filters may be arranged on the photoelectric conversion element 10a according to the embodiment of the present invention to separate the colors.

### [Optical sensor]

Examples of another application of the photoelectric conversion element include a photoelectric cell and an optical sensor, but the photoelectric conversion element according to the embodiment of the present invention is preferably used as the optical sensor. As the optical sensor, the photoelectric conversion element may be used alone, and a line sensor in which the photoelectric conversion elements are linearly arranged or a two-dimensional sensor in which the photoelectric conversion elements are arranged on a plane may be used.

### Examples

Hereinafter, the present invention will be described in more detail with reference to Examples. Materials, usage amounts, proportions, processing contents, and processing procedures shown in the following Examples can be appropriately changed without departing from the spirit of the present invention. Therefore, the scope of the present invention will not be restrictively interpreted by the following Examples.

### [Synthesis example of compound represented by Formula (1)]

Hereinafter, a synthesis method of the compound represented by Formula (1) will be shown by taking a synthesis example of a compound (D-6) as an example.

### <Synthesis of compound (D-6)>

The compound (D-6) was synthesized according to the following scheme.

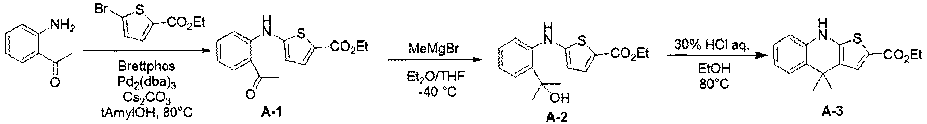

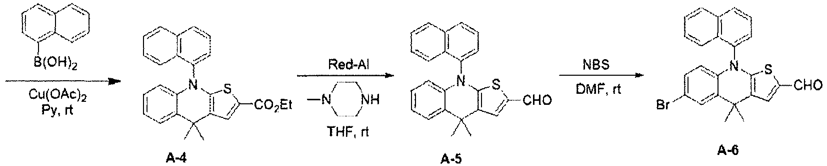

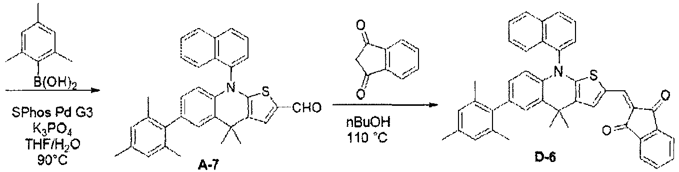

Under a nitrogen atmosphere, to a flask were added 2-aminoacetophenone (66.0 g, 489 mmol), ethyl 5-bromothiophene-2-carboxylate (115 g, 489 mmol), cesium carbonate (239 g, 734 mmol), 2-(dicyclohexylphosphino)-3,6-dimethoxy-2',4',6'-triisopropyl-1,1'-biphenyl (6.56 g, 12.2 mmol), tris(dibenzylideneacetone) dipalladium (5.6 g, 6.1 mmol), and tert-amyl alcohol (490 mL), and the obtained reaction solution was reacted at 80°C for 3 hours. After the reaction solution was allowed to cool, ethyl acetate (450 mL) was added, the mixture was filtered through celite, and then the obtained filtrate was concentrated to obtain a crude product. Hexane (300 mL) was added to the obtained crude product, and the resulting solid was filtered. The obtained solid was purified by a short column (eluent: hexane/ethyl acetate = 3/1). Hexane (800 mL) was added to the obtained crude product, the mixture was stirred at room temperature for 40 minutes, and then collection by filtration is performed to obtain a compound A-1 (98 g, yield of 69%).

Under a nitrogen atmosphere, the compound A-1 (108 g, 373 mmol) and tetrahydrofuran (620 mL) were placed in a flask, and cooled to -40°C. Subsequently, a 3.0 M methyl magnesium bromide-diethyl ether solution (497 mL, 1492 mmol) was added dropwise to the solution over 30 minutes, and then a reaction was performed for 1.5 hours. The obtained reaction solution was poured into a liquid mixture of an aqueous ammonium chloride solution (2L) and ethyl acetate (1.5 L), and a pH was adjusted to 5 with acetic acid. The organic phase was collected, the water phase was extracted once with ethyl acetate, and then the collected organic phase was washed with saturated saline. The obtained solution was dried over sodium sulfate and then filtered, and the obtained filtrate was concentrated to obtain a crude product. The obtained crude product was purified by silica gel chromatography (eluent: hexane/ethyl acetate = 9/1 to 7/3) to obtain a compound A-2 (54.6 g, yield of 48%).

The compound A-2 (88 g, 290 mmol), ethanol (880 mmol), and 30 mass% hydrochloric acid (440 mmol) were sequentially added to a flask, and stirred at 80°C for 1.5 hours. The obtained reaction solution was poured into a liquid mixture of water (2 L) and ethyl acetate (2 L), and the organic phase was collected. The water phase was extracted with ethyl acetate, and then the collected organic phase was washed with saturated saline. The obtained solution was dried over sodium sulfate and then filtered, and the obtained filtrate was concentrated to obtain a crude product. Hexane (300 mL) was added to the obtained crude product, and the resultant was stirred at 50°C for 2.5 hours. The obtained solid was collected by filtration to obtain a compound A-3 (66.3 g, yield of 80%).

To a flask were added 1-naphthylboronic acid (6.88 g, 40 mmol), copper acetate (3.63 g, 20 mmol), pyridine (57 mL), the compound A-3 (5.75 g, 20 mmol), and the mixture was reacted at room temperature for 8 hours. Ethyl acetate (300 mL) was added to the obtained reaction solution, and then a pH was adjusted to 1 with 3 N hydrochloric acid. The organic phase was collected, the water phase was extracted once with ethyl acetate, and then the collected organic phase was washed with saturated saline. The obtained solution was dried over sodium sulfate and then filtered, and the obtained filtrate was concentrated to obtain a crude product. The obtained crude product was purified by the silica gel chromatography (eluent: hexane/ethyl acetate = 100/0 to 75/25) to obtain a compound A-4 (3.27 g, yield of 40%).

Under a nitrogen atmosphere, a solution (19.4 mL, 70 mmol) of sodium bis(2-methoxyethoxy)aluminium hydride and 3.6 M toluene, and tetrahydrofuran (20 mL) were added to a flask and cooled to -20°C, and then N-methylpiperazine (7.77 mL, 70 mmol) was added dropwise. After 15 minutes, the temperature was raised to room temperature, followed by stirring for 45 minutes. Potassium tert-butoxide (783 mg, 7.0 mmol) was added to the obtained reaction solution, and the mixture was stirred at room temperature for 15 minutes. The obtained suspension was added dropwise to a solution of the compound A-4 (7.1 g, 17.2 mmol) in tetrahydrofuran (15 mL) at -10°C, and the mixture was reacted for 1 hour. The obtained reaction solution was added dropwise to a liquid mixture of ethyl acetate and 1 N aqueous sodium hydroxide solution, and the organic phase was collected. The water phase was extracted twice with ethyl acetate, and then the collected organic phase was washed with water and saturated saline. The obtained solution was dried over sodium sulfate and then filtered, and the obtained filtrate was concentrated to obtain a crude product. The obtained crude product was purified by the silica gel chromatography (eluent: hexane/ethyl acetate = 9/1 to 7/3) to obtain a compound A-5 (4.73 g, yield of 75%).

The compound A-5 (4.7 g, 12.7 mmol), N,N-dimethylformamide (50 mL), and N-bromosuccinimide (2.7 g, 15.3 mmol) were sequentially added to a flask, and reacted at room temperature for 4 hours. The obtained reaction solution was poured into water (500 mL), and the resulting solid was collected by filtration. The obtained solid was dissolved in ethyl acetate and washed with water and saturated saline. The obtained solution was dried over sodium sulfate and then filtered, and the obtained filtrate was concentrated to obtain a crude product. The obtained crude product was purified by the silica gel chromatography (eluent: hexane/ethyl acetate = 9/1 to 7/3) to obtain a compound A-6 (5.53 g, yield of 97%).

Under a nitrogen atmosphere, the compound A-6 (5.5 g, 12.3 mmol), 2,4,6-trimethylphenylboronic acid (6.0 g, 36.8 mmol), potassium phosphate (13.0 g, 61.4 mmol), 2-dicyclohexylphosphino-(2',6'-dimethoxybiphenyl)[2-(2'-amino-1,1'-biphenyl)]palladium(II ) methanesulfonate (0.95 g, 1.22 mmol), tetrahydrofuran (50 mL), and water (10 mL) were added to a flask, and reacted at 90°C for 2.5 hours. Ethyl acetate and water were added to the obtained reaction solution, and the organic phase was collected. The water phase was extracted with ethyl acetate, and then the collected organic phase was washed with water and saturated saline. The obtained solution was dried over sodium sulfate and then filtered, and the obtained filtrate was concentrated to obtain a crude product. The obtained crude product was purified by the silica gel chromatography (eluent: hexane/ethyl acetate = 9/1 to 7/3) to obtain a compound A-7 (5.55 g, yield of 93%).

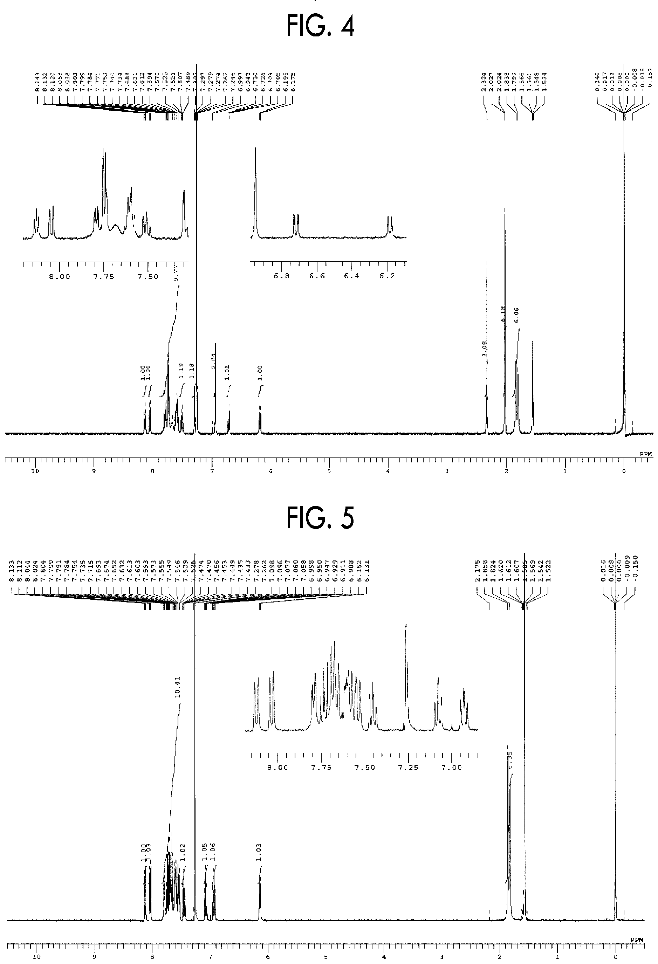

The compound A-7 (5.5 g, 11.3 mmol), 1,3-indandione (1.8 g, 12.4 mmol), and n-butanol (70 mL) were sequentially added to a flask, and reacted at 110°C for 6 hours. A solvent of the obtained reaction solution was distilled off to obtain a crude product. The obtained crude product was dissolved in benzene chloride at 70°C and then allowed to cool to room temperature. To the obtained suspension was added methanol in an amount which is twice that of the benzene chloride, and the resulting solid was collected by filtration. Furthermore, the obtained solid was recrystallized from methylene chloride-methanol (1:2, 300 mL) to obtain a compound D-6 (6.07 g, yield of 87%). The obtained compound (D-6) was identified by nuclear magnetic resonance (NMR) and mass spectrometry (MS).
¹H NMR spectrum (400 MHz, CDCl₃) is shown in Fig. 4.
MS(ESI⁺)m/z: 616.3 ([M + H]⁺)

Compounds (D-1) to (D-17) shown in Examples were synthesized with reference to the synthesis method of the compound (D-6).

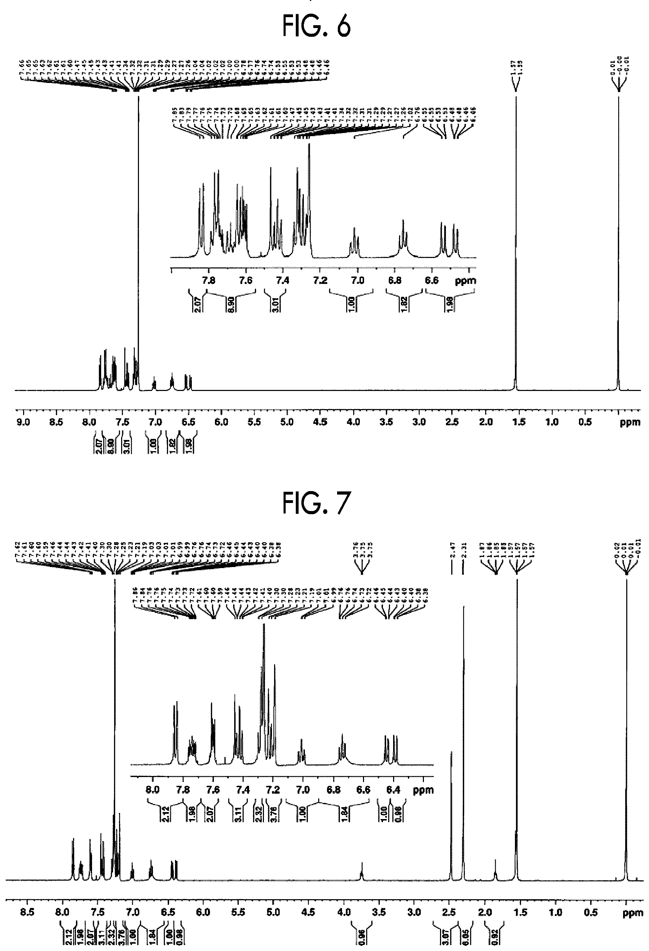

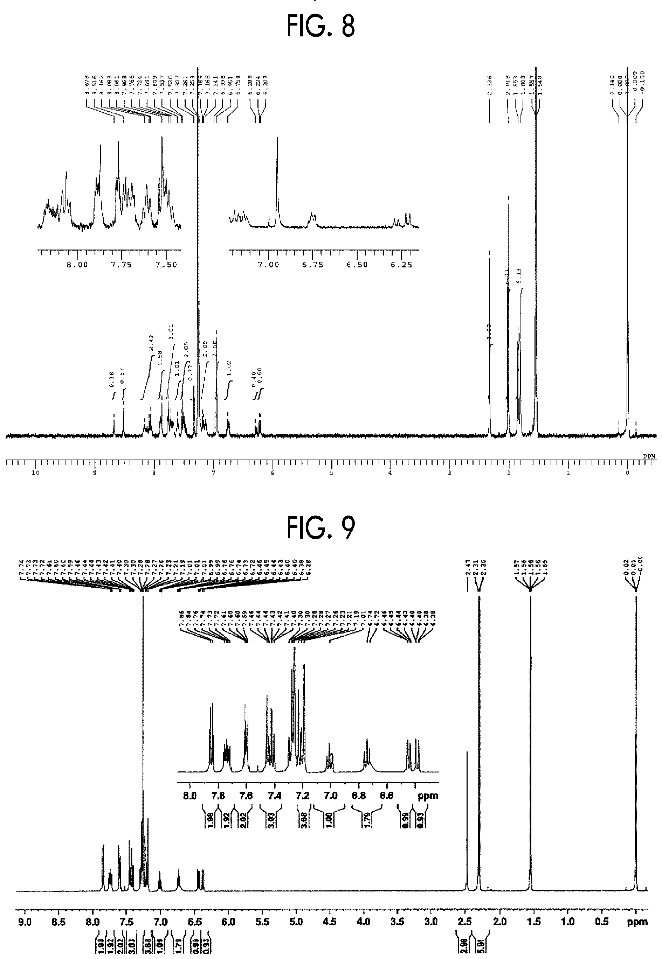

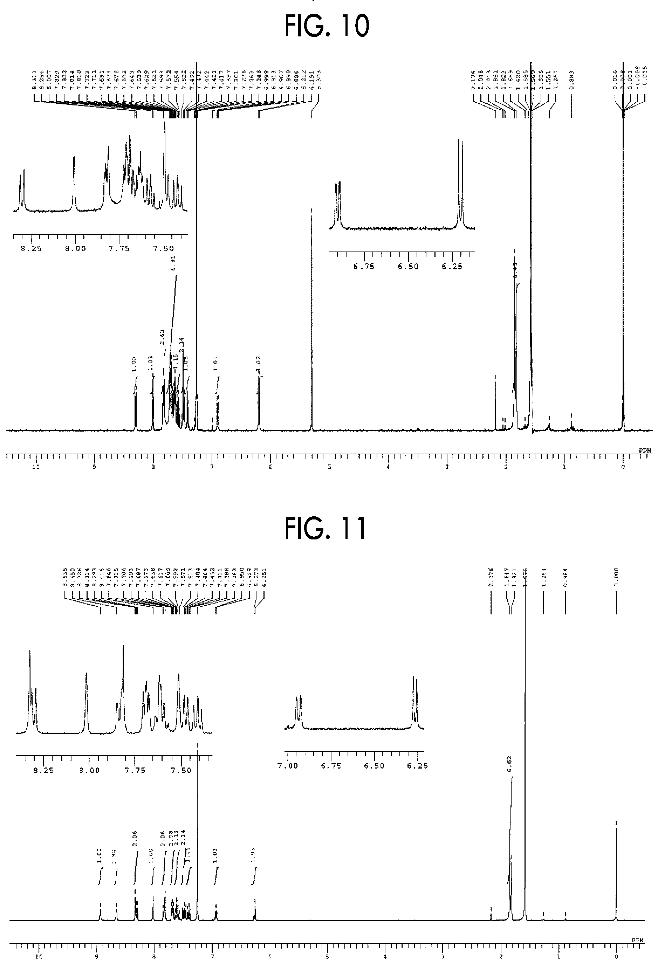

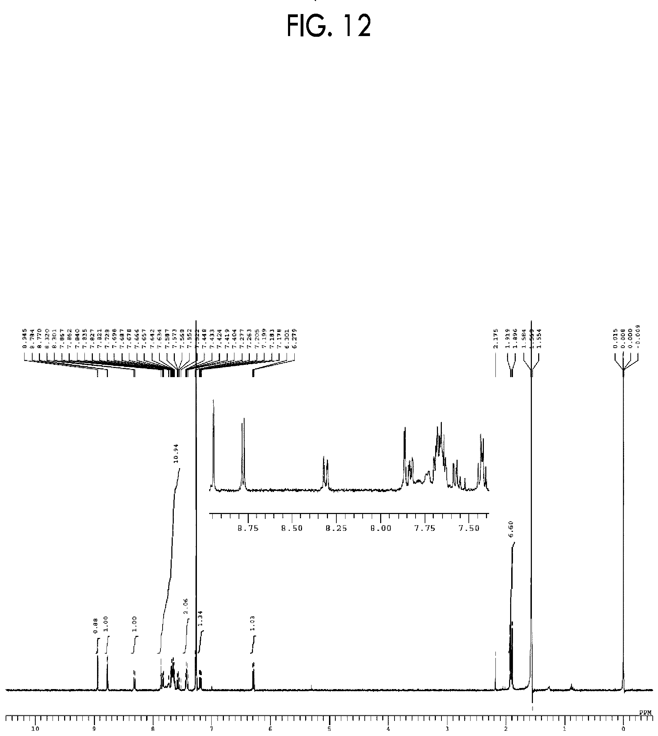

¹H NMR spectra (400 MHz, CDCl₃) of the compounds (D-2), (D-4), (D-7) to (D-9), (D-12), (D-13), and (D-15) are shown in Figs. 5 to 12, respectively.

Structures of the compounds (D-1) to (D-17) and a comparative compound (R-1) are shown below.

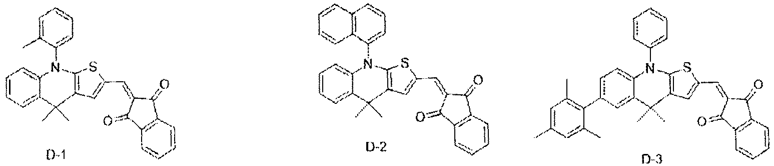

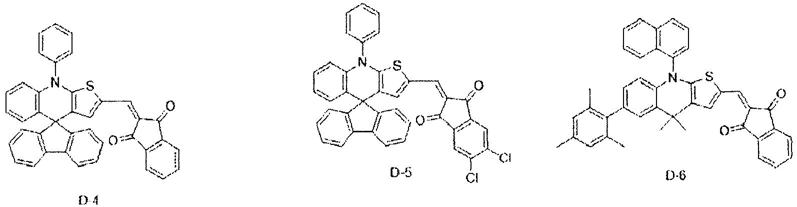

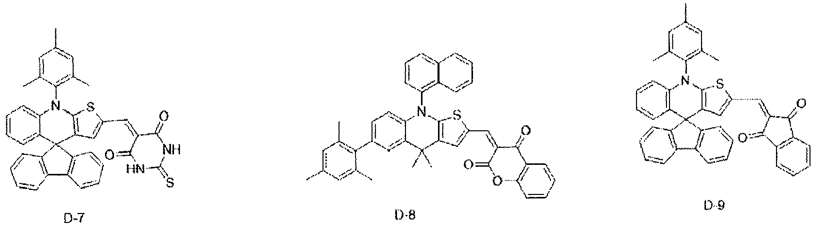

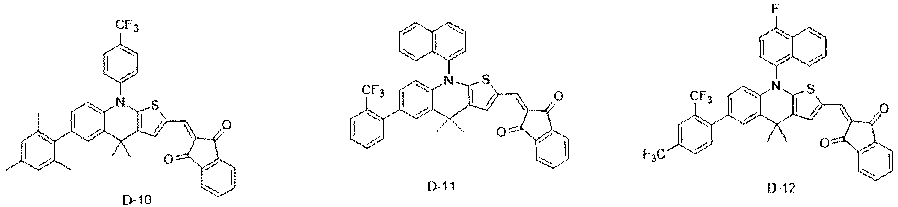

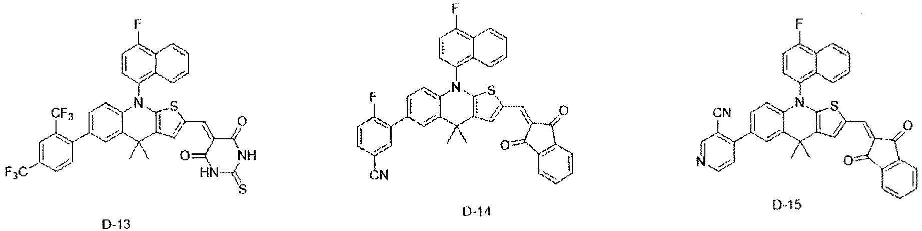

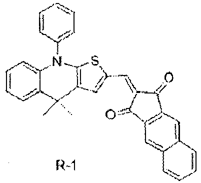

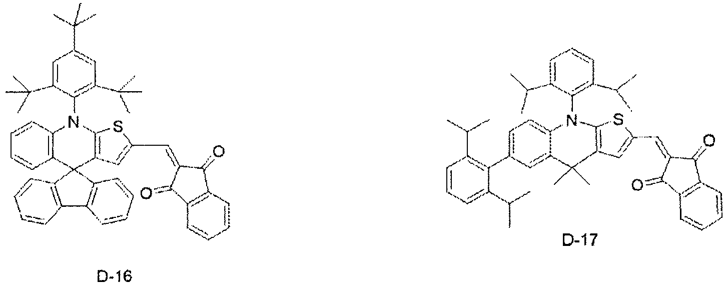

### [Various evaluations]

### <Production of vapor-deposited film>

In a state where the temperature of a glass substrate was controlled to 25°C, each of the compounds (D-1 to D-17 and R-1) obtained by the vacuum deposition method was vapor-deposited to form a film, and thus a vapor-deposited film having a thickness of 100 nm was formed on the glass substrate.

### <Measurement of absorption waveform of vapor-deposited film>

The absorption shape of the obtained vapor-deposited film was measured using a spectrophotometer U3310 manufactured by Hitachi High-Tech Corporation. The absorption maximum wavelength of the obtained absorption spectrum, and the width (the absorption half-width) at an absorbance of 0.5 in a case where an absorbance at the absorption maximum is normalized to 1 are shown in Table 1.

### <Production of photoelectric conversion element>

A photoelectric conversion element having a form shown in Fig. 1 was produced using the obtained compound. Here, the photoelectric conversion element consists of the lower electrode 11, the electron blocking film 16A, the photoelectric conversion film 12, and the upper electrode 15.

Specifically, an amorphous ITO was formed into a film on a glass substrate by the sputtering method to form the lower electrode 11 (a thickness: 30 nm), and the following compound (EB-1) was formed into a film on the lower electrode 11 by a vacuum heating vapor deposition method to form the electron blocking film 16A (a thickness: 30 nm).

Furthermore, in a state where the temperature of the substrate was controlled to 25°C, the compound (D-1) and the fullerene (C₆₀) were subjected to co-vapor deposition on the electron blocking film 16A by the vacuum deposition method so as to be respectively 100 nm and 50 nm in terms of a single layer to form a film, and thus the photoelectric conversion film 12 having the bulk hetero structure of 150 nm was formed.

In addition, the amorphous ITO was formed into a film on the photoelectric conversion film 12 by a sputtering method to form the upper electrode 15 (the transparent conductive film) (a thickness: 10 nm). After a SiO film was formed as the sealing layer on the upper electrode 15 by the vacuum deposition method, an aluminum oxide (Al₂O₃) layer was formed thereon by an atomic layer chemical vapor deposition (ALCVD) method to produce a photoelectric conversion element.

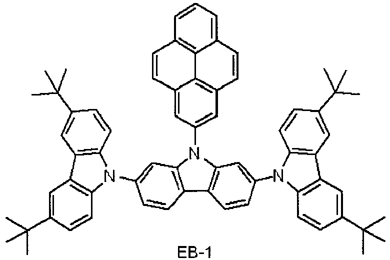

Similarly, the photoelectric conversion elements were produced using the compounds (D-2) to (D-17) and (R-1).

### <<Checking of driving (evaluation of photoelectric conversion efficiency (external quantum efficiency))>>

The driving of each of the obtained photoelectric conversion elements was checked. A voltage was applied to each photoelectric conversion element so that the electric field strength was 2.0 × 10⁵ V/cm. Thereafter, light was irradiated from the upper electrode (the transparent conductive film) side, the photoelectric conversion efficiency (the external quantum efficiency) at 540 nm was measured, and as a result, it was confirmed that the photoelectric conversion elements produced using the compounds (D-1) to (D-17) and (R-1) all exhibited photoelectric conversion efficiency of 60% or greater and had external quantum efficiency sufficient as a photoelectric conversion element. The external quantum efficiency was measured using a constant energy quantum efficiency measuring device manufactured by NIHON OPTEL CORPORATION. An irradiation light amount was 50 µW/cm².

### <<Evaluation of heat resistance>>

Heat resistance of each of the obtained photoelectric conversion elements was evaluated. Specifically, each of the obtained photoelectric conversion elements was heated on a hot plate at 190°C for 30 minutes. A voltage was applied to heated each photoelectric conversion element so that the electric field strength was 2.0 × 10⁵ V/cm, light was irradiated from the upper electrode (the transparent conductive film) side, and the photoelectric conversion efficiency (the external quantum efficiency) at 540 nm was measured. The external quantum efficiency was measured using a constant energy quantum efficiency measuring device manufactured by NIHON OPTEL CORPORATION. The evaluation was performed by using a relative value of the photoelectric conversion efficiency after heating in a case where the photoelectric conversion efficiency before heating was 1. A case where the relative value is 0.95 or greater was evaluated as A, a case where the relative value is 0.90 or greater and less than 0.95 was evaluated as B, and a case where the relative value is less than 0.90 was evaluated as C. Furthermore, practically, B or higher is preferable and A is more preferable. The results are shown in Table 1.

In addition, in Table 1, the column of "Corresponding to Formula (3)" is A in a case where each compound corresponds to the compound represented by Formula (3), and "B" in a case where each compound does not correspond to the compound represented by Formula (3).

In Table 1, the column of "Corresponding to Formula (3A)" is A in a case where each compound corresponds to the compound represented by Formula (3A), and "B" in a case where each compound does not correspond to the compound represented by Formula (3A).

In Table 1, the column of "Corresponding to Formula (2A)" is A in a case where each compound corresponds to the compound represented by Formula (2A), and "B" in a case where each compound does not correspond to the compound represented by Formula (2A).

In Table 1, "-" in the column of "Corresponding to Formula (4A), Formula (4B), or Formula (5)" in the column of "Ar¹" means a case where Ar¹ does not correspond to any of the group represented by Formula (4A), the group represented by Formula (4B), or the group represented by Formula (5).

In Table 1, "-" in the column of "Corresponding to Formula (4A), Formula (4B), or Formula (5)" in the column of "R⁴" means a case where R⁴ does not correspond to any of the group represented by Formula (4A), the group represented by Formula (4B), or the group represented by Formula (5).

In Table 1, "-" in the column of "Corresponding to Formula (4A), Formula (4B), or Formula (5)" in the column of "R³, R⁵, and R⁶" means a case where R³, R⁵, and R⁶ each do not correspond to any of the group represented by Formula (4A), the group represented by Formula (4B), or the group represented by Formula (5).

**[Table 1]**

| Compound | Corresponding to Formula (3) | Corresponding to Formula (3A) | Corresponding to Formula (2A) | Ar¹ | | R³ to R⁶ | | | Absorption maximum wavelength (nm) | Absorption half-width (nm) | Heat resistance | Note |
|---|---|---|---|---|---|---|---|---|---|---|---|---|
| | | | | | | R⁴ | | R³, R⁵, and R⁶ | | | | |
| | | | | Corresponding to Formula (4A), Formula (4B), or Formula (5) | In case of Formula (4A), the number of substituents among R^{c1} to R^{c4} or in case of Formula (4B), the number of substituents R^{c12} | Corresponding to Formula (4A), Formula (4B), or Formula (5) | In case of Formula (4A), the number of substituents among R^{c1} to R^{c4} or in case of Formula (4B), the number of substituents R^{c12} | Corresponding to Formula (4A), Formula (4B), or Formula (5) | | | | |
| D-1 | A | B | B | Corresponding to Formula (4A) | 0 | - | - | - | 548 | 95 | B | Example |
| D-2 | A | B | B | Corresponding to Formula (5) | - | - | - | - | 548 | 92 | A | Example |
| D-3 | A | B | B | - | - | Corresponding to Formula (4A) | 2 | - | 544 | 92 | A | Example |
| D-4 | A | A | A | - | - | - | - | - | 544 | 79 | A | Example |
| D-5 | A | A | A | - | - | - | - | - | 563 | 83 | A | Example |
| D-6 | A | B | B | Corresponding to Formula (5) | - | Corresponding to Formula (4A) | 2 | - | 545 | 86 | A | Example |
| D-7 | B | B | A | Corresponding to Formula (4A) | 2 | - | - | - | 549 | 77 | B | Example |
| D-8 | B | B | B | Corresponding to Formula (5) | - | Corresponding to Formula (4A) | 2 | - | 553 | 84 | B | Example |
| D-9 | A | A | A | Corresponding to Formula (4A) | 2 | - | - | - | 541 | 74 | A | Example |
| D-10 | A | B | B | | - | Corresponding to Formula (4A) | 2 | - | 534 | 90 | A | Example |
| D-11 | A | B | B | Corresponding to Formula (5) | - | Corresponding to Formula (4A) | 0 | - | 543 | 77 | A | Example |
| 3D-12 | A | B | B | Corresponding to Formula (5) | - | Corresponding to Formula (4A) | 1 | - | 536 | 83 | A | Example |
| D-13 | B | B | B | Corresponding to Formula (5) | - | Corresponding to Formula (4A) | 1 | - | 548 | 88 | B | Example |
| D-14 | A | B | B | Corresponding to Formula (5) | - | Corresponding to Formula (4A) | 1 | - | 543 | 86 | A | Example |
| D-15 | A | B | B | Corresponding to Formula (5) | - | Corresponding to Formula (4B) | 0 | - | 543 | 86 | A | Example |
| D-16 | A | A | A | Corresponding to Formula (4A) | 2 | | - | - | 542 | 72 | A | Example |
| D-17 | A | B | B | Corresponding to Formula (4A) | 1 | Corresponding to Formula (4A) | 1 | - | 541 | 80 | A | Example |
| R-1 | B | B | B | - | - | - | - | - | 573 | 101 | C | Comparative Example |

As shown in Table 1, it was confirmed that in the photoelectric conversion element according to the embodiment of the present invention, the absorption half-width of the photoelectric conversion film was narrower and the heat resistance of the photoelectric conversion element was superior, compared to Comparative Example.

In addition, from the comparison of the compound D-1, the compound D-2, and the compound D-3, it was confirmed that in a case where at least one of Ar¹, R³, ..., or R⁶ in the compound represented by Formula (3) represents the group represented by Formula (5) (the compound D-2 corresponds to this case), or in a case where at least one of Ar¹, R³, ..., or R⁶ in the compound represented by Formula (3) represents the group represented by Formula (4A) and at least one (preferably, at least two) of R^{e1}, ..., or R^{e4} in the group represented by Formula (4A) represents a substituent (the compound D-3 corresponds to this case), the absorption half-width of the photoelectric conversion film was narrower and the heat resistance of the photoelectric conversion element was superior.

From the comparison of the compound D-1 to the compound D-3, and the compound D-6, the compound D-8, the compound D-10 to the compound D-15, and the compound D-17, it was confirmed that in a case where the compound corresponds to Formula (2), and in the compound, Ar¹ represents the group represented by Formula (4A), the group represented by Formula (4B), or the group represented by Formula (5), and the aromatic ring represented by B¹ has the group represented by Formula (4A) or the group represented by Formula (4B) as a substituent (the compound D-6, the compound D-8, the compound D-11 to the compound D-15, and the compound D-17 correspond to this case), the absorption half-width of the photoelectric conversion film was narrower.

From the comparison of the compound D-6 and the compound D-8, and the comparison of the compound D-12 and the compound D-13, it was confirmed that in a case where the compound corresponds to Formula (3) (the compound D-6 and the compound D-12 correspond to this case), the heat resistance of the photoelectric conversion element was superior.

From the comparison of the compound D-1 to the compound D-3, and the compound D-4, the compound D-5, the compound D-7, the compound D-9, and the compound D-16, it was confirmed that in a case where the compound corresponds to Formula (2A) or Formula (3A), the absorption half-width of the photoelectric conversion film was narrower (the compound D-4, the compound D-5, the compound D-7, the compound D-9, and the compound D-16 correspond to this case). From the comparison of the compound D-4, the compound D-5, the compound D-7, the compound D-9, and the compound D-16, it was confirmed that, in particular, in a case where the compound corresponds Formula (3A) (the compound D-4, the compound D-5, the compound D-9, and the compound D-16), the heat resistance of the photoelectric conversion element was superior.

From the results of Examples, it was confirmed that in a case where the compound corresponds to any of the following [1] or [2], the absorption half-width of the photoelectric conversion film was narrower and the heat resistance of the photoelectric conversion element was superior.
[1] The specific compound is a compound represented by Formula (3), and in the compound, Ar¹ represents the group represented by Formula (4A), the group represented by Formula (4B), or the group represented by Formula (5) and at least one of R³, ..., or R⁶ represents the group represented by Formula (4A) or the group represented by Formula (4B) (the compound D-6, the compound D-11, the compound D-12, the compound D-14, the compound D-15, and the compound D-17 correspond to this case).
[2] The specific compound is a compound represented by Formula (3A) (the compound D-4, the compound D-5, the compound D-9, and the compound D-16 correspond to this case).

### [Production of imaging element]

Using the compounds (D-1) to (D-17), imaging elements each having a form similar to that shown in Fig. 3 were produced.

The photoelectric conversion element functioning as a green photoelectric conversion element was produced by the method described above.

Furthermore, the blue photoelectric conversion element and the red photoelectric conversion element were produced with reference to the description in JP2005-303266A.

Since, in the obtained imaging element, the photoelectric conversion film in the photoelectric conversion element according to the embodiment of the present invention had a narrow half-width of the absorption peak, light was easily received by the blue photoelectric conversion element and the red photoelectric conversion element and the color separation performance was excellent.

### Explanation of References

- 10a, 10b:: Photoelectric conversion element
- 11:: Conductive film (lower electrode)
- 12:: Photoelectric conversion film
- 15:: Transparent conductive film (upper electrode)
- 16A:: Electron blocking film
- 16B:: Positive hole blocking film
- 20a:: Imaging element
- 22:: Blue photoelectric conversion element
- 24:: Red photoelectric conversion element

## Claims

1. A photoelectric conversion element comprising, in the following order:
a conductive film;
a photoelectric conversion film; and
a transparent conductive film,
wherein the photoelectric conversion film contains a compound represented by Formula (1), 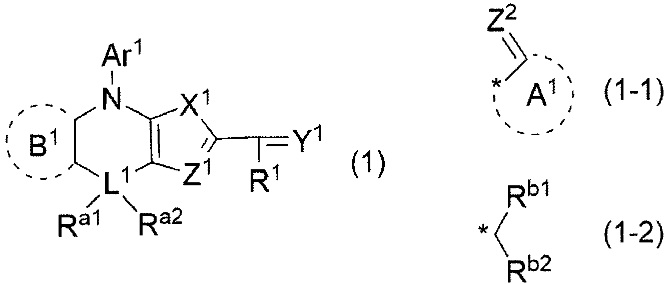
in Formula (1), Ar¹ represents an aryl group which may have a substituent or a heteroaryl group which may have a substituent, R¹ represents a hydrogen atom or a substituent, X¹ represents a sulfur atom, an oxygen atom, a selenium atom, a tellurium atom, S(=O), S(=O)₂, NR^{c1}, SiR^{c2}R^{c3}, or CR^{c4}R^{c5}, R^{c1} to R^{c5} each independently represent a hydrogen atom or a substituent, Z¹ represents CR^{c6} or a nitrogen atom, R^{c6} represents a hydrogen atom or a substituent, L¹ represents a carbon atom, a silicon atom, or a germanium atom, R^{a1} and R^{a2} each independently represent a hydrogen atom or a substituent, R^{a1} and R^{a2} may be bonded to each other to form a ring, B¹ represents an aromatic ring which may have a substituent, Y¹ represents a group represented by Formula (1-1) or a group represented by Formula (1-2), A¹ represents a ring containing at least two carbon atoms, Z² represents an oxygen atom, a sulfur atom, =NR^{Z1}, or =CR^{Z2}R^{Z3}, R^{Z1} represents a hydrogen atom or a substituent, R^{Z2} and R^{Z3} each independently represent a cyano group or -COOR^{Z4}, R²⁴ represents an alkyl group which may have a substituent, an aryl group which may have a substituent, or a heteroaryl group which may have a substituent, R^{b1} and R^{b2} each independently represent a cyano group or -COOR^{c7}, R^{c7} represents an alkyl group or an aryl group, and * represents a bonding position,
provided that in a case where both R^{a1} and R^{a2} are selected from the group consisting of a hydrogen atom and a methyl group, the compound represented by Formula (1) satisfies the following condition (1a) or the following condition (1b),
Condition (1a): Ar¹ represents a group represented by Formula (1-3), and
Condition (1b): B¹ represents an aromatic ring having a substituent selected from the group consisting of an alkyl group, a silyl group, and a group represented by Formula (1-3), and 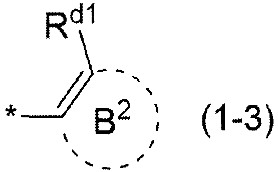
in Formula (1-3), B² represents an aromatic ring which may have a substituent, R^{d1} represents a hydrogen atom or a substituent selected from the group consisting of an alkyl group, a silyl group, an alkoxy group, an alkylthio group, a cyano group, a halogen atom, an aryl group, a heteroaryl group, an alkenyl group, and an alkynyl group, substituents that R^{d1} and B² have may be bonded to each other to form a non-aromatic ring, and * represents a bonding position,
provided that in a case where B² represents a benzene ring which may have a substituent, R^{d1} represents a substituent selected from the group consisting of an alkyl group, a silyl group, an alkoxy group, an alkylthio group, a cyano group, a halogen atom, an aryl group, a heteroaryl group, an alkenyl group, and an alkynyl group.

2. The photoelectric conversion element according to claim 1,
wherein the compound represented by Formula (1) is a compound represented by Formula (2), 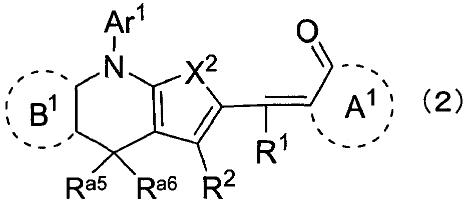 in Formula (2), Ar¹ represents an aryl group which may have a substituent or a heteroaryl group which may have a substituent, A¹ represents a ring containing at least two carbon atoms, R¹ and R² each independently represent a hydrogen atom or a substituent, X² represents a sulfur atom, an oxygen atom, or a selenium atom, R^{a5} and R^{a6} each independently represent a hydrogen atom, an alkyl group, an aryl group, or a heteroaryl group, R^{a5} and R^{a6} may be bonded to each other to form a ring, and B¹ represents an aromatic ring which may have a substituent,
provided that in a case where both R^{a5} and R^{a6} are selected from the group consisting of a hydrogen atom and a methyl group, the compound represented by Formula (2) satisfies the following condition (2a) or the following condition (2b),
Condition (2a): Ar¹ represents a group represented by Formula (1-3), and
Condition (2b): B¹ represents an aromatic ring having a substituent selected from the group consisting of an alkyl group, a silyl group, and a group represented by Formula (1-3).

3. The photoelectric conversion element according to claim 2,
wherein R^{a5} and R^{a6} each independently represent an alkyl group having 3 or more carbon atoms, an aryl group, or a heteroaryl group.

4. The photoelectric conversion element according to claim 2 or 3,
wherein the compound represented by Formula (2) is a compound represented by Formula (2A), 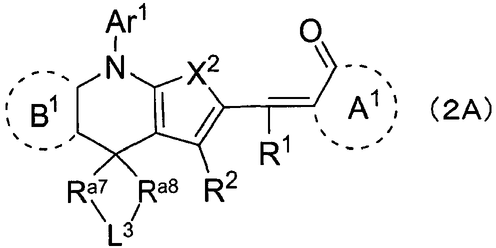 in Formula (2A), Ar¹ represents an aryl group which may have a substituent or a heteroaryl group which may have a substituent, A¹ represents a ring containing at least two carbon atoms, R¹ and R² each independently represent a hydrogen atom or a substituent, X² represents a sulfur atom, an oxygen atom, or a selenium atom, R^{a7} and R^{a8} each independently represent an arylene group or a heteroarylene group, L³ represents a single bond or a divalent linking group, and B¹ represents an aromatic ring which may have a substituent.

5. The photoelectric conversion element according to claim 2,
wherein the compound represented by Formula (2) is a compound represented by Formula (3), 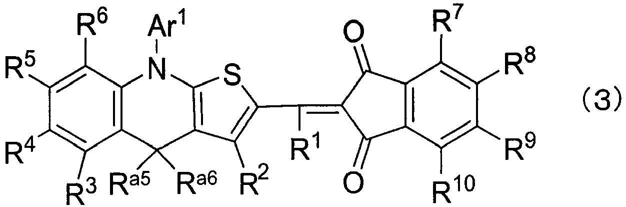 in Formula (3), Ar¹ represents an aryl group which may have a substituent or a heteroaryl group which may have a substituent, R¹ to R¹⁰ each independently represent a hydrogen atom or a substituent, R^{a5} and R^{a6} each independently represent a hydrogen atom, an alkyl group, an aryl group, or a heteroaryl group, and R^{a5} and R^{a6} may be bonded to each other to form a ring,
provided that in a case where both R^{a5} and R^{a6} are selected from the group consisting of a hydrogen atom and a methyl group, the compound represented by Formula (3) satisfies the following condition (3a) or the following condition (3b),
Condition (3a): Ar¹ represents a group represented by Formula (1-3), and
Condition (3b): at least one of R³, ..., or R⁶ represents a group selected from the group consisting of an alkyl group, a silyl group, and a group represented by Formula (1-3).

6. The photoelectric conversion element according to claim 5,
wherein R^{a5} and R^{a6} each independently represent an alkyl group having 3 or more carbon atoms, an aryl group, or a heteroaryl group.

7. The photoelectric conversion element according to claim 5 or 6,
wherein the compound represented by Formula (3) is a compound represented by Formula (3A), 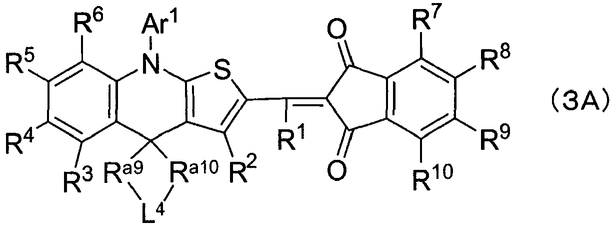 in Formula (3A), Ar¹ represents an aryl group which may have a substituent or a heteroaryl group which may have a substituent, R¹ to R¹⁰ each independently represent a hydrogen atom or a substituent, R^{a9} and R^{a10} each independently represent an arylene group or a heteroarylene group, and L⁴ represents a single bond or a divalent linking group.

8. The photoelectric conversion element according to any one of claims 2 to 4,
wherein B¹ represents an aromatic ring having a substituent, and
at least one of the substituent that B¹ has or Ar¹ represents a group represented by Formula (4A), a group represented by Formula (4B), or a group represented by Formula (5), 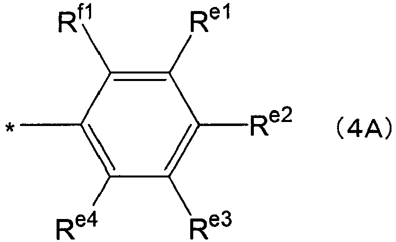 in Formula (4A), R^{e1} to R^{e4} each independently represent a hydrogen atom or a substituent, R^{e1} to R^{e4} may be bonded to each other to form a ring, and R^{f1} represents an alkyl group, a cyano group, or a halogen atom, 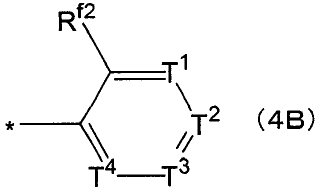 in Formula (4B), T¹ to T⁴ each independently represent CR^{e12} or a nitrogen atom, R^{e12} represents a hydrogen atom or a substituent, and R^{f2} represents an alkyl group, a cyano group, or a halogen atom,
provided that at least one of T¹, ..., or T⁴ represents a nitrogen atom, and
in a case where a plurality of R^{e12,}s are present in Formula (4B), R^{e12,}s may be the same as or different from each other, and R^{e12,}s may be bonded to each other to form a ring, and 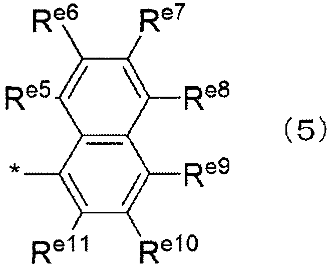 in Formula (5), R^{e5} to R^{e11} each independently represent a hydrogen atom or a substituent, and R^{e5} to R^{e11} may be bonded to each other to form a ring.

9. The photoelectric conversion element according to claim 8,
wherein at least one of R^{e1}, ..., or R^{e4} in the group represented by Formula (4A) represents a substituent.

10. The photoelectric conversion element according to claim 8 or 9,
wherein Ar¹ represents the group represented by Formula (4A), the group represented by Formula (4B), or the group represented by Formula (5), and at least one of the substituents that B¹ has represents the group represented by Formula (4A) or the group represented by Formula (4B).

11. The photoelectric conversion element according to any one of claims 5 to 7,
wherein at least one of Ar¹, R³, ..., or R⁶ represents a group represented by Formula (4A), a group represented by Formula (4B), or a group represented by Formula (5), 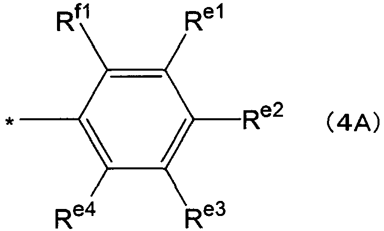 in Formula (4A), R^{e1} to R^{e4} each independently represent a hydrogen atom or a substituent, R^{e1} to R^{e4} may be bonded to each other to form a ring, and R^{f1} represents an alkyl group, a cyano group, or a halogen atom, 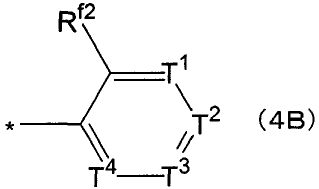 in Formula (4B), T¹ to T⁴ each independently represent CR^{e12} or a nitrogen atom, R^{e12} represents a hydrogen atom or a substituent, and R^{f2} represents an alkyl group, a cyano group, or a halogen atom,
provided that at least one of T¹, ..., or T⁴ represents a nitrogen atom, and
in a case where a plurality of R^{e12,}s are present in Formula (4B), R^{e12,}s may be the same as or different from each other, and R^{e12}'s may be bonded to each other to form a ring, and 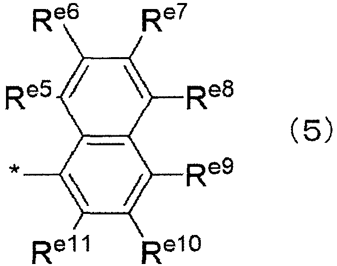 in Formula (5), R^{e5} to R^{e11} each independently represent a hydrogen atom or a substituent, and R^{e5} to R^{e11} may be bonded to each other to form a ring.

12. The photoelectric conversion element according to claim 11,
wherein at least one of R^{e1}, ..., or R^{e4} in the group represented by Formula (4A) represents a substituent.

13. The photoelectric conversion element according to claim 11 or 12,
wherein Ar¹ represents the group represented by Formula (4A), the group represented by Formula (4B), or the group represented by Formula (5), and at least one of R³, ..., or R⁶ represents the group represented by Formula (4A) or the group represented by Formula (4B).

14. The photoelectric conversion element according to any one of claims 1 to 13,
wherein the photoelectric conversion film further contains an n-type organic semiconductor, and
the photoelectric conversion film has a bulk hetero structure formed in a state where the compound represented by Formula (1) and the n-type organic semiconductor are mixed.

15. The photoelectric conversion element according to claim 14,
wherein the n-type organic semiconductor contains fullerenes selected from the group consisting of a fullerene and a derivative thereof.

16. The photoelectric conversion element according to any one of claims 1 to 15, further comprising:
one or more interlayers between the conductive film and the transparent conductive film, in addition to the photoelectric conversion film.

17. An imaging element comprising:
the photoelectric conversion element according to any one of claims 1 to 16.

18. The imaging element according to claim 17, further comprising:
another photoelectric conversion element which receives light having a wavelength different from a wavelength of light received by the photoelectric conversion element.

19. The imaging element according to claim 18,
wherein the photoelectric conversion element and the other photoelectric conversion element are laminated, and
at least some of incidence rays are transmitted through the photoelectric conversion element, and then are received by the other photoelectric conversion element.

20. The imaging element according to claim 18 or 19,
wherein the photoelectric conversion element is a green photoelectric conversion element, and
the other photoelectric conversion element includes a blue photoelectric conversion element and a red photoelectric conversion element.

21. An optical sensor comprising:
the photoelectric conversion element according to any one of claims 1 to 16.

22. A compound represented by Formula (1), 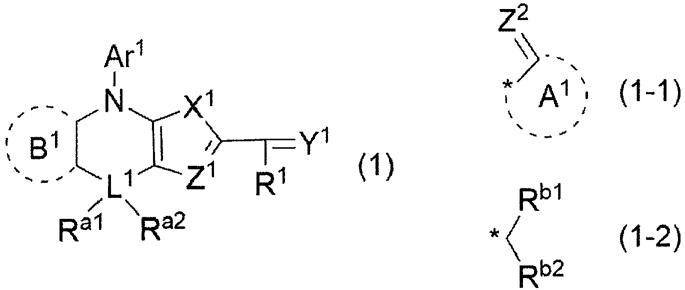
in Formula (1), Ar¹ represents an aryl group which may have a substituent or a heteroaryl group which may have a substituent, R¹ represents a hydrogen atom or a substituent, X¹ represents a sulfur atom, an oxygen atom, a selenium atom, a tellurium atom, S(=O), S(=O)₂, NR^{c1}, SiR^{c2}R^{c3}, or CR^{c4}R^{c5}, R^{c1} to R^{c5} each independently represent a hydrogen atom or a substituent, Z¹ represents CR^{c6} or a nitrogen atom, R^{c6} represents a hydrogen atom or a substituent, L¹ represents a carbon atom, a silicon atom, or a germanium atom, R^{a1} and R^{a2} each independently represent a hydrogen atom or a substituent, R^{a1} and R^{a2} may be bonded to each other to form a ring, B¹ represents an aromatic ring which may have a substituent, Y¹ represents a group represented by Formula (1-1) or a group represented by Formula (1-2), A¹ represents a ring containing at least two carbon atoms, Z² represents an oxygen atom, a sulfur atom, =NR^{Z1}, or =CR^{Z2}R^{Z3}, R^{Z1} represents a hydrogen atom or a substituent, R^{Z2} and R^{Z3} each independently represent a cyano group or -COOR^{Z4}, R^{Z4} represents an alkyl group which may have a substituent, an aryl group which may have a substituent, or a heteroaryl group which may have a substituent, R^{b1} and R^{b2} each independently represent a cyano group or -COOR^{c7}, R^{c7} represents an alkyl group or an aryl group, and * represents a bonding position,
provided that in a case where both R^{a1} and R^{a2} are selected from the group consisting of a hydrogen atom and a methyl group, the compound represented by Formula (1) satisfies the following condition (1a) or the following condition (1b),
Condition (1a): Ar¹ represents a group represented by Formula (1-3), and
Condition (1b): B¹ represents an aromatic ring having a substituent selected from the group consisting of an alkyl group, a silyl group, and a group represented by Formula (1-3), and 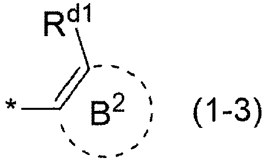 in Formula (1-3), B² represents an aromatic ring which may have a substituent, R¹ represents a hydrogen atom or a substituent selected from the group consisting of an alkyl group, a silyl group, an alkoxy group, an alkylthio group, a cyano group, a halogen atom, an aryl group, a heteroaryl group, an alkenyl group, and an alkynyl group, substituents that R^{d1} and B² have may be bonded to each other to form a non-aromatic ring, and * represents a bonding position,
provided that in a case where B² represents a benzene ring which may have a substituent, R^{d1} represents a substituent selected from the group consisting of an alkyl group, a silyl group, an alkoxy group, an alkylthio group, a cyano group, a halogen atom, an aryl group, a heteroaryl group, an alkenyl group, and an alkynyl group.

23. The compound according to claim 22,
wherein the compound represented by Formula (1) is a compound represented by Formula (3), 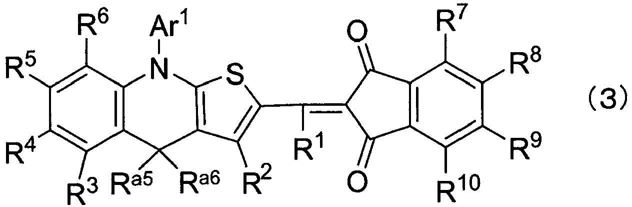 in Formula (3), Ar¹ represents an aryl group which may have a substituent or a heteroaryl group which may have a substituent, R¹ to R¹⁰ each independently represent a hydrogen atom or a substituent, R^{a5} and R^{a6} each independently represent a hydrogen atom, an alkyl group, an aryl group, or a heteroaryl group, and R^{a5} and R^{a6} may be bonded to each other to form a ring,
provided that in a case where both R^{a5} and R^{a6} are selected from the group consisting of a hydrogen atom and a methyl group, the compound represented by Formula (3) satisfies the following condition (3a) or the following condition (3b),
Condition (3a): Ar¹ represents a group represented by Formula (1-3), and
Condition (3b): at least one of R³, ..., or R⁶ represents a group selected from the group consisting of an alkyl group, a silyl group, and a group represented by Formula (1-3).
